# EUROPEAN PATENT SPECIFICATION

(11) **EP 3 946 391 B1**
(45) Date of publication and mention of the grant of the patent: **25.03.2026**
(21) Application number: 20782777.5
(22) Date of filing: 31.03.2020
(51) Int. Cl.: A61K 35/74, A61K 35/744, A61K 35/742, A61K 35/741, A61K 35/747, A61K 39/395, A61K 39/00, C07K 16/28, A61P 35/00

(54) **MICROBIAL CONSORTIUM AND USES THEREOF**
MIKROBIELLES KONSORTIUM UND VERWENDUNGEN DAVON
CONSORTIUM MICROBIEN ET SES UTILISATIONS

(30) Priority: 31.03.2019 IL 26573519
(43) Date of publication of application: 09.02.2022
(73) Proprietor: Biomica Ltd., Rehovot 76121 (IL)
(72) Inventor: HABER, Elran, 5545049 Kiryat Ono (IL); MESHNER, Shiri, 4339419 Raanana (IL); ESHAR, Shiri, 7684200 Emek Sorek (IL); TIROSH, Osnat, 7400434 Ness-Ziona (IL); KRUGER BEN SHABAT, Sheerli, 4373315 Raanana (IL); RINGEL, Yehuda, 6912524 Tel Aviv (IL)
(74) Representative: J A Kemp LLP
(86) International application number: PCT/IL2020/050389
(87) International publication number: WO 2020/202148

(56) References cited:
- EP-A1- 1 917 869
- EP-A2- 3 223 834
- WO-A1-2017/089794
- WO-A1-2021/137237
- WO-A2-2016/086205
- WO-A2-2018/136617
- GB-A- 2 587 735
- US-A1- 2016 354 416
- US-A1- 2018 071 344
- YUILLE SAMANTHA ET AL: "Human gut bacteria as potent class I histone deacetylase inhibitors in vitro through production of butyric acid and valeric acid", PLOS ONE, PUBLIC LIBRARY OF SCIENCE, US, vol. 13, no. 7, 1 January 2018 (2018-01-01), pages 1 - 2, XP009508155, ISSN: 1932-6203, DOI: 10.1371/JOURNAL.PONE.0201073
- ZITVOGEL, LAURENCE ET AL.: "The microbiome in cancer immunotherapy: Diagnostic tools and therapeutic strategies", SCIENCE, vol. 359.6382, 2018, pages 1366 - 1370, XP055746988
- SCHWABE, ROBERT F. ET AL.: "The microbiome and cancer", NATURE REVIEWS CANCER, vol. 13.11, 2013, pages 800 - 812, XP055261349

## Description

### TECHNOLOGICAL FIELD

The present discourse relates to consortium of microorganisms and uses thereof, for example, for treating cancer.

### BACKGROUND ART

References considered to be relevant as background to the presently disclosed subject matter are listed below:
[1] Lynch, Susan V., and Oluf Pedersen. "The human intestinal microbiome in health and disease." New England Journal of Medicine 375.24 (2016): 2369-2379.
[2] International application publication No. WO2016086208.
[3] International application publication No. WO2017218680.
[4] International application publication No. WO2018064165.
[5] International application publication No. WO2019046646.
[6] Takeshi Tanoue et al. "A defined commensal consortium elicits CD8 T cells and anti-cancer immunity." Nature 565 (2019): 600-605.
[7] US application publication No. US2018/071344 discloses probiotic and prebiotic compositions, and methods of use thereof for treatment and prevention of graft versus host disease.
[8] Yuille, Samantha et al. "Human gut bacteria as potent class I histone deacetylase inhibitors in vitro through production of butyric acid and valeric acid." PLoS ONE (2018).
[9] GB application publication No. GB2587735 discloses compositions comprising bacterial strains.
[10] International application publication No. WO2016086205 discloses probiotic and prebiotic compositions, and methods of use thereof for modulation of the microbiome.
[11] European application publication No. EP3223834 discloses probiotic and prebiotic compositions comprising blautia species for reducing inflammation.
**[12]** European application publication No. EP1917869 discloses probiotic/non-probiotic combinations.
[13] US application publication No. US2016354416 discloses treatment of cancer by manipulation of commensal microflora.
[14] Zitvogel, Laurence et al. "The microbiome in cancer immunotherapy: Diagnostic tools and therapeutic strategies." Science 359.6382 (2018): 1366-1370
[15] Schwabe, Robert F et al. "The microbiome and cancer." Nature Rev Cancer 13.11 (2013): 800-812
[16] International application publication No. WO2017089794 discloses compositions comprising bacterial strains.
[17] International application publication No. WO2018136617 discloses methods of treating cancer.
[18] International application publication No. WO2021137237 discloses a microbial consortium and uses thereof.

Acknowledgement of the above references herein is not to be inferred as meaning that these are in any way relevant to the patentability of the presently disclosed subject matter.

### BACKGROUND

The human microbiome is a diverse multi species population of more than trillion microorganisms including bacteria, fungi, archaea, and viruses that collectively play an important role in various physiological process within a host affecting human health and disease. For example, it was shown that the microbiome has the ability to increase energy extraction from food, to serve as a physical barrier to protect the host from external pathogens and to assist in the development of the host immune system [1].

Compositions of bacterial strains, and their use for treating disease were described [**2-6**].

### GENERAL DESCRIPTION

The present invention is defined by the appended claims. The technical information set out below may in some respects go beyond the disclosure of the invention defined by the appended claims. The additional technical information is provided to place the invention in a broader technical context and to illustrate possible related technical developments. Such additional technical information which does not fall within the scope of the appended claims is not part of the invention. Accordingly, any embodiments and examples of the present disclosure which do not fall under the scope of the appended claims do not form part of the invention and are merely provided for illustrative purposes.

The invention relates to a microbial consortium comprising two or more microorganisms for use in a method of inducing a pro-inflammatory effect and treating cancer in a human subject, wherein said two or more microorganisms are selected from *Oenococcus oeni* species, *Clostridium cellulovorans* species, *Cetobacterium somerae* species, *Corynebacterium glyciniphilum* species *and Clostridium tyrobutyricum* species, further wherein the cancer is selected from breast cancer, lung cancer, pancreatic cancer, bladder cancer, melanoma, kidney cancer, head and neck cancer, and lymphoma, and further wherein the microbial consortium is administered in combination with a checkpoint inhibitor that is an anti- PD-1 inhibitor.

### BRIEF DESCRIPTION OF THE DRAWINGS

In order to better understand the subject matter that is disclosed herein and to exemplify how it may be carried out in practice, embodiments will now be described, by way of non-limiting example only, with reference to the accompanying drawings, in which:

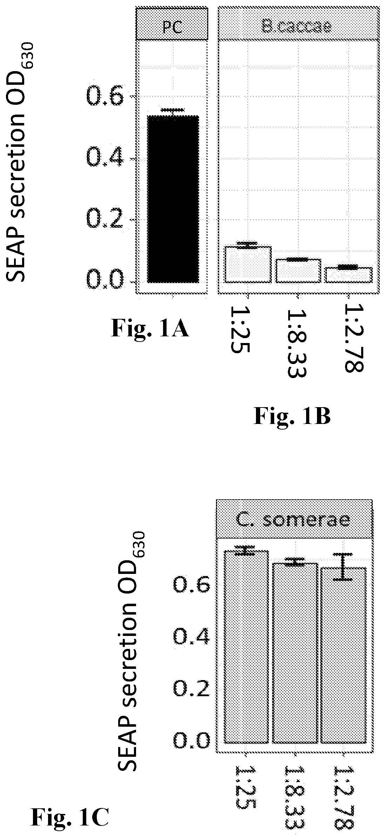

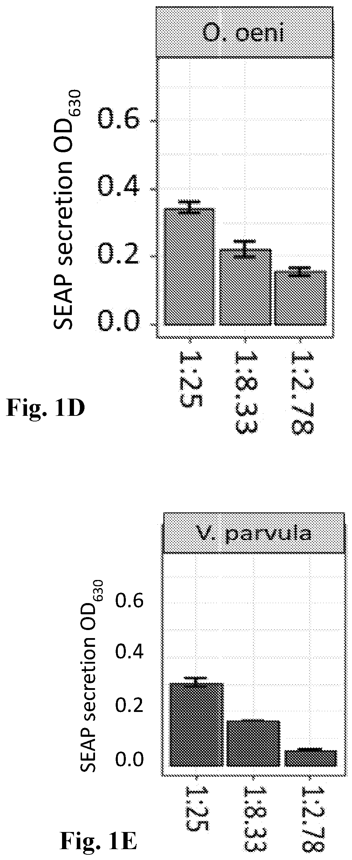

**Figs. 1A -1E** are bar graphs showing the Toll-like receptor 4 (TLR4) activation by bacterial strains in RAW cells at various bacteria:cells ratios; lipopolysaccharide (LPS) as positive control (PC) **(****Fig. 1A****),** *Bacteroides caccae* CL03T12C61 **(****Fig. 1B****),** *Cetobacterium somerae* ATCC BAA-474 **(****Fig. 1C****),** *Oenococcus oeni* PSU-1 **(****Fig. 1D****),** *Veillonella parvula ATCC 17745* **(****Fig. 1E****),** results are represented as means ±SEM.

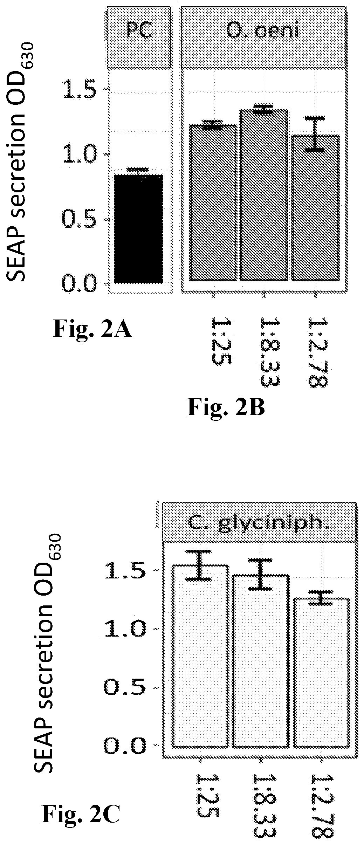

**Figs. 2A-2C** are bar graphs showing the Toll-like receptor 2 (TLR2) activation by bacterial strains in RAW cells at various bacteria:cells ratios; LPS as positive control **(****Fig. 2A****),** *Oenococcus oeni* PSU-1 **(****Fig. 2B****),** *Corynebacterium glyciniphilum* AJ 3170 **(****Fig. 2C****),** results are represented as means ±SEM.

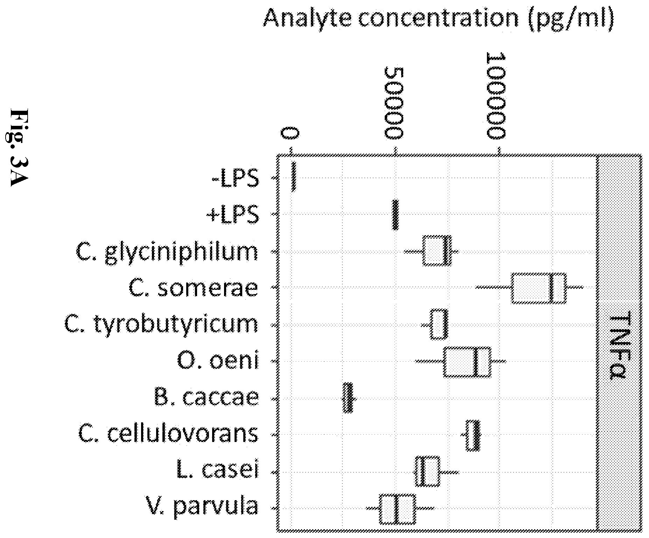

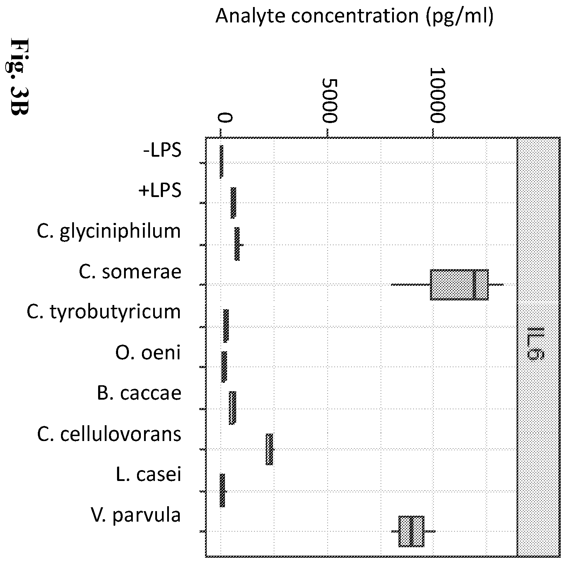

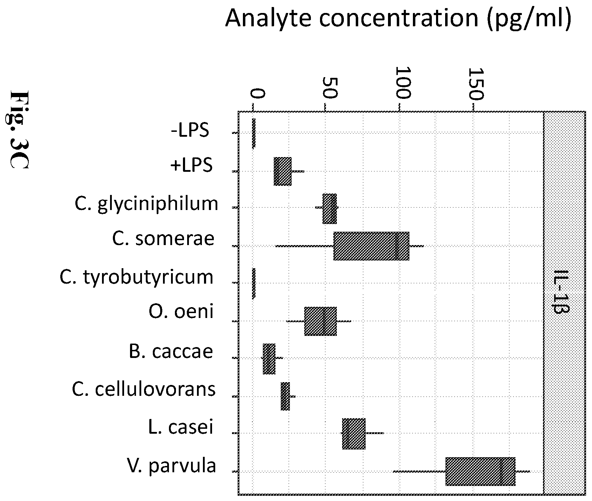

**Figs. 3A-3C** are boxplots showing the efficacy of different bacterial strains in stimulating cytokines' secretion from RAW cells, TNFα **(****Fig. 3A****),** IL6 **(****Fig. 3B****),** IL1β **(****Fig. 3C****),** results are provided as median with the interquartile range, and error bars are the 1.5 times interquartile range (whiskers).

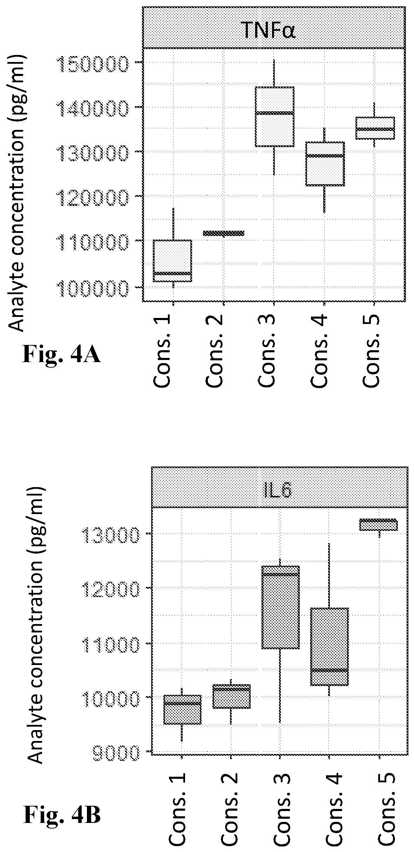

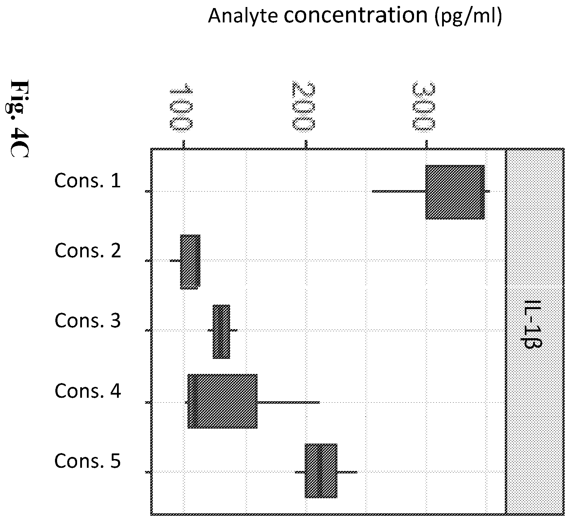

**Figs. 4A-4C** are boxplots showing the efficacy of different combinations of bacterial strains (Cons. 1, Cons. 2, Cons. 3, Cons. 4, Cons. 5) in stimulating cytokines' secretion from RAW cells, TNFα **(****Fig. 4A****),** IL6 **(****Fig. 4B****),** IL1β **(****Fig. 4C****),** the total concentration of all bacteria in each consortium was 1X10^8 CFU/ml, results are provided as median with the interquartile range, and error bars are the 1.5 times interquartile range (whiskers).

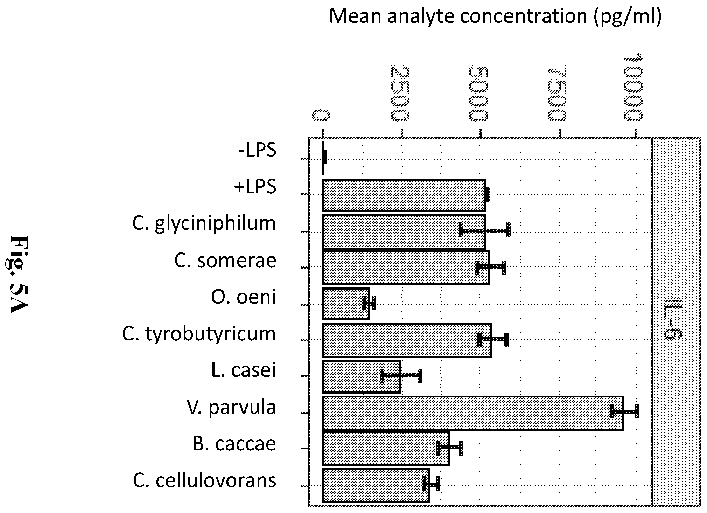

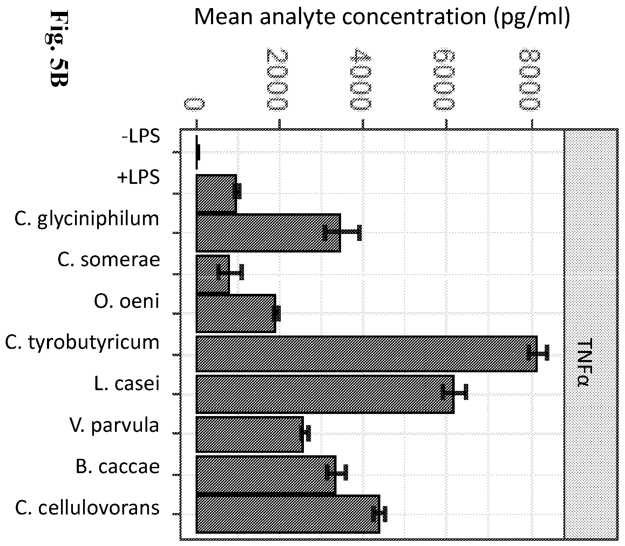

**Figs. 5A** and **5B** are bar graphs showing the efficacy of bacterial strains in stimulating cytokines' secretion from human peripheral blood mononuclear cell (PBMCs), IL6 **(****Fig. 5A****),** TNFα **(****Fig. 5B****),** the concentration of each bacterial strain is 1X10^8 CFU/ml; results are represented as means ±SEM.

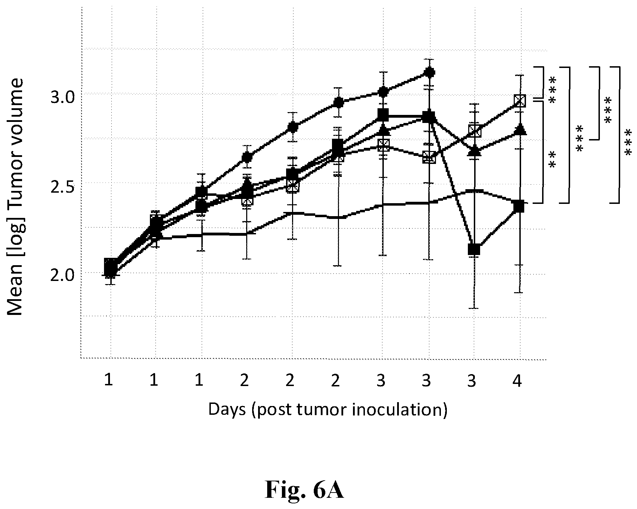

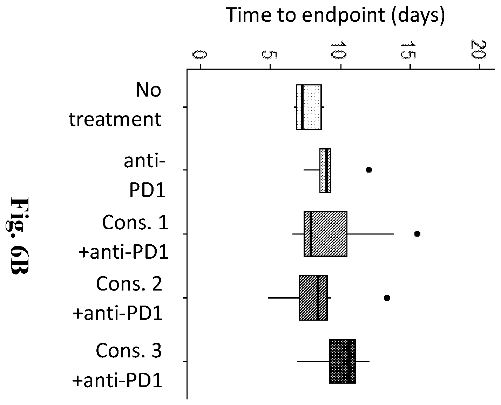

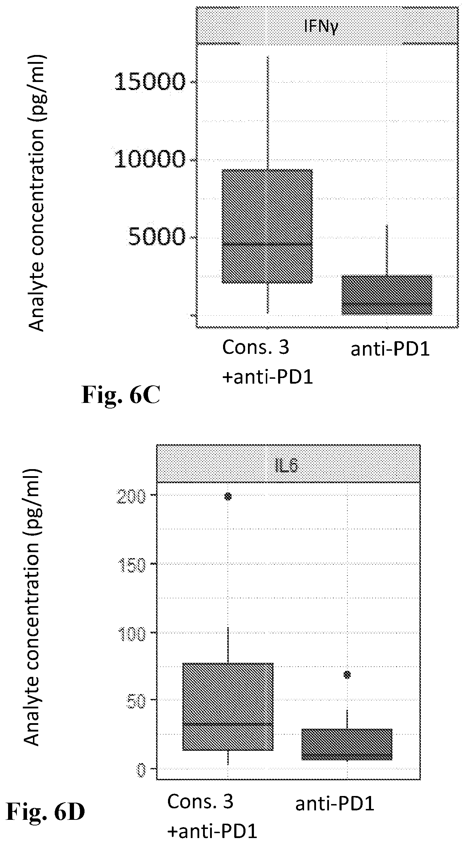

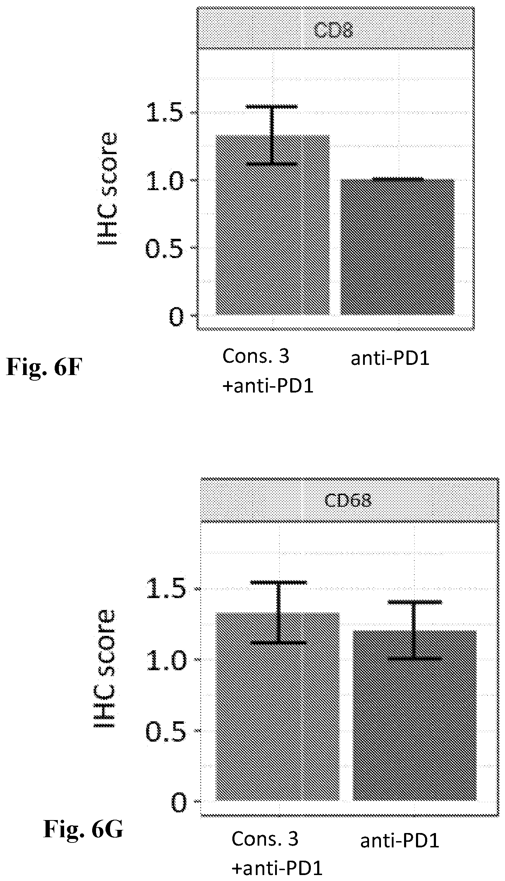

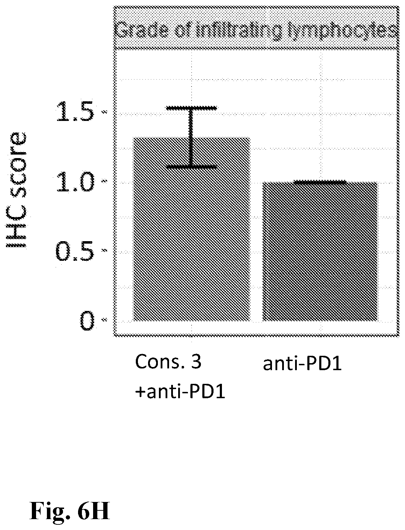

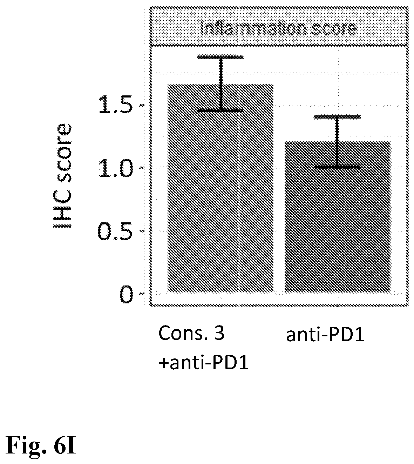

**Figs. 6A** to **6I** show *in vivo* effect of bacterial combinations (Cons. 1, Cons. 2, Cons. 3) on the response to immune checkpoint therapy for the treatment of breast cancer in a mouse model, **Fig. 6A** shows inhibition of tumor growth in bacterial combination-treated mice in addition to anti-PD1 therapy compared to anti-PD1 only as measured by log10 of tumor volumes along time, circle - no treatment, triangle - Cons. 1+anti PD-1, rectangular - Cons. 2+anti PD-1, plus sign - Cons. 3+anti PD-1, X - anti -PD1, (**p<0.01, ***p<0.001); **Fig. 6B** is a graph showing the time to reach study end-point (calculated from day 29 of the study) (tumor volume of 1000 mm²) for the various treatments; **Fig. 6C** to **6E** are boxplots showing the levels of various cytokines in the plasma of mice following treatment, **Fig. 6F** to **6I** are graphs showing immunohistochemistry analysis of expression of specific cell surface markers and inflammation scores from H&E staining of 4µm sections from paraffin-embedded dissected tumors from mice following treatment.

### DETAILED DESCRIPTION OF EMBODIMENTS

The gut microbiome includes a large number of diverse microorganisms that are capable of affecting a variety of physiological process within a host, including for example development and differentiation, activation or suppression of the host's immune system.

The present disclosure is based on a possible connection between the immune system and cancer therapy and is aimed at using specific microorganisms detected in, isolated from or purified from a microbiome, for example the gut microbiome, that contribute to modulation of the immune system and hence may be used in inhibiting cancer growth and in treatment of cancer.

To that end, the inventors have used an array of computational tools utilized to process high-throughput sequencing data and obtained high resolution detection and annotation of microbial genes and pathways as well as microbial taxa. This resulted in a mechanistic understanding of a relationship between an organisms' (such as a microorganism) and various human cellular processes related to cancer and immune function. During the analysis process several methods such as stringent statistical comparative analyses were applied to enable the identification and selection of specific microorganisms and microbial functions that differentiate between cancer patients that were found to respond to immune checkpoint blockade treatment and cancer patients that were found to be non-responders to this treatment.

The inventors envisaged that a unique and specific combination of microorganisms identified, may be used as for treating proliferative disorders. The inventors have thus suggested that the computationally identified microorganisms may be administered, alone or preferably in specific combinations, to a subject in order to enrich the microbiome diversity, induce a pro-inflammatory effective immune response and to treat cancer. The inventors further suggested that the identified microorganisms may be used for diagnostic and prognostic purposes, for example for assessing responsiveness of a subject to treatment and for determining treatment protocols.

As such, the present disclosure provides a microbial consortium. The microbial consortium comprises two or more microorganisms that are collectively capable of affecting different functional capabilities, by modulating a number of processes both in the microorganism *per se* and in the host. Such process may be for example production of short chain fatty acids (SCFAs), activation of dendritic cells, and innate immune activation.

Surprisingly, some of the identified microorganisms were found to be present at low amounts in the human microbiome and were further found to play a major role in immune mediated processes in a host. As shown in the examples below, these microorganisms, although present at low amounts in the human microbiome, elicit a profound pro-inflammatory response alone and in combinations both in *in vitro* and in *in vivo* experiments.

Thus, the present disclosure provides a microbial consortium comprising two or more microorganisms, at least one of the two or more microorganisms is present at low-abundance in a microbiome of a reference human subject.

The *microbial consortium* as used herein refers to a mixture/cocktail of microorganisms, including at least one of a bacterium and/or an archaea. When referring to at least one microorganism it should be understood as referring to one microorganism species and/or strain as classified under common scientific classification. The microorganisms being the subject of the present disclosure are present in the human microbiome and thus can be isolated and/or purified from any microbiome, such as the human microbiome, by any known method in the art as also detailed below or purified from a biological material (e.g., fecal materials, such as feces or materials isolated from the various segments of the small and large intestines). As such, the term *microorganism* used herein can refer to at least one of an isolated microorganism, a purified microorganism or a recombinant microorganism.

The microbial consortium may comprise isolated microorganisms. The microbial consortium may comprise purified microorganisms. The microbial consortium may comprise recombinant microorganisms. The microbial consortium may comprise isolated microorganisms, purified microorganisms or any combination thereof. The microbial consortium may comprise isolated microorganisms, purified microorganisms, recombinant microorganisms or any combination thereof.

It should be noted that the recombinant microorganisms are microorganisms whose genetic makeup has been altered by deliberate introduction of new genetic elements. The recombinant microorganisms may maintain the functions (cellular processes) of the original microorganism.

The microorganism may be at least one of a live microorganism or provided as spores, heat-killed, non-living form of the microorganism, or an extract or a component of the organism.

Without being bound by theory, it was suggested by the inventors that the selected/identified microorganisms are capable of treating cancer via modulation of multiple pathways, such as altering the host microbiota or modulating the production of various biological molecules (e.g., SCFA).

The attribution of the microorganisms to each one of the consortiums was done by considering the multiple functions of each one of the microorganisms and the combined functions, in order to avoid adverse effect such as, for example, damage to the intestinal epithelium. The inventors have suggested that the selected microorganisms, while capable of inducing a pro-inflammatory effect and inhibiting cancer progression, would maintain the integrity of the gut barrier. This may be achieved, for example by affecting mucin degradation or modulation of SCFAs in the gut.

The at least two or more microorganisms in the microbial consortium may possess various biological relationship such that, for example, at least one microorganism may benefit from the at least one other microorganism. For example, the metabolic products of one microorganism may be used as a substrate by another member of the consortium or by additional gut commensals, hence increasing the likelihood of intestinal colonization by the microbial consortium or promoting a desired activity of one or more microorganism. Hence, it was suggested by the inventors, that the combination of at least two microorganisms in the microbial consortium may achieve actions resulting in immune modulation through several underlying, overlapping and complementary mechanisms. For example, at least one microorganism in the microbial consortium may be capable of modulating at least one process, at times at least two processes and even at times at least three or at least four process as detailed herein below.

As described herein, at least one microorganism in the microbial consortium is present at low-abundance in a microbiome of a reference human subject. *Abundance* in the context of the present disclosure refers to a representation of the relative amount (quantity) of a specific microorganism in the microbiome. This amount can be obtained, by any method known in the art. For example, various molecular-based methods are available to characterize and quantitate the intestinal microbiota such as traditional clone libraries; direct sequencing using next-generation parallel sequencing technology; denaturing gradient gel electrophoresis and temperature gradient gel electrophoresis; terminal restriction fragment length polymorphism analysis; fluorescent in situ hybridization; and quantitative Polymerase Chain Reaction (PCR). In addition, computational analysis of sequence data including information on isolated intestinal microorganisms can be used, for example by counting the number of mapped reads to a reference genome. Using computational tools, relative abundance for each microorganism in the microbiome can be determined and is defined as the number of reads mapped to a reference genome divided by the total number of microbial reads within a given microbiome sample and normalized in methods known in the art (e.g. genome size).

The term *low-abundance* as used herein refer to a microorganism that is present in the microbiome of a reference subject in an amount that is below a predetermined standard value/s or cutoff value/s. The predetermined standard value/s or cutoff value/s may be determined as an average value for a large, heterogonous cohort of subjects or alternatively be determined for a specific population of reference subjects. The reference human subject may be a healthy subject. A healthy subject is a subject that is not diagnosed with a disease to be treated using the microbial consortium. The reference human subject may be a subject diagnosed with at least one disease, optionally a disease that can be treated using the microbial consortium. A low abundance microorganism may have an average relative abundance of less than 0.5%, at times less than 0.4%, at times less than 0.3%, at times less than 0.2%, at times even less than 0.1%. A low abundance microorganism may have an average relative abundance of between about 0.00001% to about 0.5%, at times between about 0.0001% to about 0.4%, at times between about 0.001% to about 0.3%, at times between about 0.01% to about 0.2%, at times between about 0.01% to about 0.1% at times between about 0.05% to about 0.1%.

The at least one microorganism present at low abundance in a reference human subject may have at least one of the following (i) capable of modulating at least one SCFA and (ii) comprises at least one cell envelope component. The cell envelope component is one that is being recognized by a subject's immune system or an epithelial cell, such as intestinal epithelial cell. The at least one microorganism present at low abundance in a reference human subject may be capable of modulating lactate.

The present disclosure provides a microbial consortium comprising two or more microorganisms, at least one of the two or more microorganisms may be present at low-abundance in a microbiome of a reference human subject and having at least one of (i) capable of modulating at least one SCFA, (ii) capable of modulating lactate and (iii) comprises at least one cell envelope component being recognized by a subject's immune system or an epithelial cell, such as intestinal epithelial cell.

The microbial consortium comprises at least one other microorganism. The at least one other microorganism may have at least one of (i) capable of modulating at least one SCFA, (ii) capable of modulating lactate and (iii) comprises at least one cell envelope component being recognized by a subject's immune system or an epithelial cell, such as intestinal epithelial cell.

The present disclosure provides a microbial consortium comprising two or more microorganisms, at least one of the two or more microorganisms may be present at low-abundance in a microbiome of a reference human subject and at least one other of the two or more microorganisms having at least one of (i) capable of modulating at least one SCFA, (ii) capable of modulating lactate and (iii) comprises at least one cell envelope component being recognized by a subject's immune system or an epithelial cell, such as intestinal epithelial cell.

The at least one other microorganism may have at least one of (i) capable of modulating at least one SCFA, (ii) capable of modulating lactate and (iii) comprises at least one cell envelope component recognized by a subject's immune system being recognized by a subject's immune system or an epithelial cell, such as intestinal epithelial cell is at least one microorganism being present at low-abundance in a microbiome of a reference human subject.

The at least one other microorganism may have at least one of (i) capable of modulating at least one SCFA, (ii) capable of modulating lactate and (iii) comprises at least one cell envelope component recognized by a subject's immune system being recognized by a subject's immune system or an epithelial cell, such as intestinal epithelial cell is at least one microorganism characterized by not being present at low-abundance in a microbiome of a reference human subject.

One or more of the microorganisms, for example the at least one microorganism being present at low-abundance in a microbiome of a reference human subject, may be modulator of physiological processes, i.e. capable of modulating at least one physiological process in the microorganism and/or the host. As used herein the term *"a microorganism capable of modulating"* relates to a *microorganism* that acts directly (e.g., by binding) or indirectly on a biological entity, such as a receptor or an enzyme, and leads to a modulation of its activation.

Modulation may either activate and increase activation of a physiological/cellular process, or alternatively, decrease and inhibit activation of a physiological/cellular process, specifically, any of these processes, as specified herein. The term "modulator" therefore includes inhibitors and activators and hence the microorganisms may be considered as either inhibitors or activators of a specific process. Activators are agents that induce, activate, stimulate, increase, facilitate, enhance activation, sensitize or up regulate a physiological/cellular process, e.g., a microorganism that increase/activate a process or any indirect activator. Inhibitors are agents that inhibit, partially or totally block stimulation or activation, decrease, prevent, delay activation, inactivate, desensitize, or down regulate a physiological/cellular process, e.g., a microorganism that reduce/decrease a process or any indirect inhibitor. The term modulation may encompasses also maintaining for example expression or function. In addition and for example, the term modulate as used herein may be used as enhancing secretion of one or more substance, as described herein or maintaining the .

It should be noted that *modulation* encompass processes (pathways) within the microorganism(s) and/or within the host.

Processes that are modulated endogenous in the microorganisms, *i.e.* microbial-related processes, are all collectively denoted herein as *"'functional microbial processes.* The functional microbial processes may take place in at least one specific type (species or microorganism) microorganism (endogenous process). Such *microbial processes* produce, for example, a microorganism product that may be secreted from the microorganism to affect the host. Accordingly, the microbial consortium may, for example, modulate (by activation) a microbial process resulting in the production of SCFA or lactate and their subsequent secretion to the host.

The microorganisms identified by the inventors were found to modulate a variety of cellular processes in a host, denoted herein as *'functional host processes".* These processes may be mediated by a microbial component (i.e. produced and secreted by a microorganism) directly or indirectly via a host component. Thus, at least one of the two or more microorganisms, for example the at least one microorganism being present at low-abundance in a microbiome of a reference human subject, is capable of modulating a cellular process in a host subject (functional host process). Without being bound by theory, the inventors suggested that an interaction exists between a specific process in the microorganisms and a cellular process being activated by this specific feature in a host subject. For example, SCFA such as acetic acid, propionic acid or butyric acid, being produced by a microorganism(s) in the microbial component, may be secreted from the specific microorganism to the host gut and may in turn inhibit the host's HDACs (histone deacetylase) enzymes or activation of a surface component of an immune cell of a host subject. In addition, microbial cell envelop component (such as LPS) may activate the host's TLRs (Toll-like receptors). The host component may be, for example an enzyme. The microbial component may be produced by the microorganism and may be secreted into the host gut.

As described herein, modulation of a cellular processes may result in production of a substance. It should be noted *producing* as used herein refers to production *per se* of a substance either directly or indirectly in the microorganism, for example by at least one microorganism (for example in the microorganism) or a component thereof (such as an enzyme) or in the host by a component of a host, and also, at times, when the substance is produced within the microorganism, this may also encompasses the secretion of the specific substance from the at least one microorganism into the host's gut and stool.

As shown in examples 1 and 2 below, the identified microorganisms were capable of modulating a variety of processes, for example activating TLRs, secretion of a variety of pro-inflammatory cytokines and expression of various surface component of an immune cell. As also shown in the examples below, surprisingly, the microorganisms that are present in low abundance in the in a microbiome of a reference human subject exhibited a profound and substantive modulation (activation, secretion, expression) of a variety of processes both in macrophages and in peripheral blood mononuclear cell (PBMC). Hence, it was suggested by the inventors that the computationally identified and selected low abundance microorganisms, despite their "natural" low abundance, have the capability of eliciting a specific, profound immune response.

As described herein, the microbial consortium is capable of modulating a variety of process, including the *functional microbial processes* and/or the *functional host processes,* all referred herein collectively as *cellular processes.* The cellular process relates (encompasses) to modulation of at least one of the following (i) a Toll-like receptor (TLR), (ii) a surface component of an immune cell, (iii) nuclear factor kappa-light-chain-enhancer of activated B cells (NF-κB), (iv) a dendritic cell, (v) a pro-inflammatory cytokine and (vi) gut barrier integrity.

The microbial consortium may be capable of modulating of at least one of the following (i) a TLR, (ii) a surface component of an immune cell, (iii) NF-κB, (iv) a dendritic cell, (v) a pro-inflammatory cytokine and (vi) gut barrier integrity.

The microbial consortium may be capable of modulating at least one of the following (i) activating a TLR, (ii) activating a surface component of an immune cell, (iii) activating NF-κB, (iv) activating a dendritic cell, (v) secreting a pro-inflammatory cytokine and (vi) maintaining or enhancing gut barrier integrity.

The microbial consortium may be capable of modulating of at least one of the following (i) activating a TLR, (ii) activating a surface component of an immune cell and (iii) maintaining a pro-inflammatory cytokine

The at least one microorganism present at low abundance in a reference human subject may be capable of modulating at least one of (i) a TLR, (ii) a surface component of an immune cell, (iii) NF-κB, (iv) a dendritic cell, (v) a pro-inflammatory cytokine and (vi) gut barrier integrity.

The at least one of the two or more microorganisms, for example the at least one of the two or more microorganisms being present at low-abundance in a microbiome of a reference human subject, may be capable of modulating at least one of (i) a TLR, (ii) a surface component of an immune cell and (iii) a pro-inflammatory cytokine.

The microbial consortium be have at least one of (i) capable of modulating (activating) at least one SCFA, (ii) comprises at least one cell envelope component being recognized by a subject's immune system or an epithelial cell, such as intestinal epithelial cell, (iii) capable of modulating (activating)at least one TLR, (iv) capable of modulating (activating)a surface component of an immune cell, (v) capable of modulating (activating) NF-κB, (vi) capable of modulating (activating)a dendritic cell, (vii) capable of secreting a pro-inflammatory cytokine and (viii) capable of maintaining or enhancing a gut barrier integrity.

The microbial consortium may have at least one of (i) capable of modulating at least one SCFA, (ii) comprises at least one cell envelope component being recognized by a subject's immune system or an epithelial cell, such as intestinal epithelial cell, (iii) capable of modulating at least one TLR, (iv) capable of modulating a surface component of an immune cell, and (v) capable of modulating a pro-inflammatory cytokine.

The at least one of the two or more microorganisms, for example the at least one of the two or more microorganisms being present at low-abundance in a microbiome of a reference human subject, may have at least one of (i) capable of modulating at least one SCFA, (ii) comprises at least one cell envelope component being recognized by a subject's immune system or an epithelial cell, such as intestinal epithelial cell, (iii) capable of modulating at least one TLR, (iv) capable of modulating a surface component of an immune cell, and (v) capable of modulating a pro-inflammatory cytokine.

As described herein, the microbial consortium comprises at least one microorganism, for example the at least one of the two or more microorganisms being present at low-abundance in a microbiome of a reference human subject, that is capable of modulating/activating at least one SCFA.

SCFA that are being produced by the gut microbiome serve as an energy source to a host intestinal epithelium and regulate gut motility, inflammation, immune function, glucose homeostasis, and energy harvesting.

The SCFA may comprise at least one of an acetic acid, a propionic acid, a butyric acid, a valeric acid, an isovaleric acid, a formic acid, an isobutyric acid and any combination thereof.

The SCFA may comprise at least one of an acetic acid, a propionic acid, a butyric acid and any combination thereof.

The microbial consortium may comprise at least one microorganism, for example the at least one microorganism being present at low-abundance in a microbiome of a reference human subject, that may be capable of secreting at least one of an acetic acid, a propionic acid, a butyric acid, a valeric acid, an isovaleric acid, a formic acid, an isobutyric acid, lactate and any combination thereof.

The microbial consortium may comprise at least one microorganism, for example the at least one microorganism being present at low-abundance in a microbiome of a reference human subject, that may be capable of secreting at least one of an acetic acid, a propionic acid, a butyric acid, lactate and any combination thereof.

The microbial consortium may comprise at least one microorganism, for example the at least one microorganism being present at low-abundance in a microbiome of a reference human subject, that comprises at least one cell envelope component.

The term *cell envelope component* refers to a structural/ chemical entity that reside on or embedded in the microorganism's membrane or cell wall or outer membrane. The cell envelope components may have an ability to be recognized by a subject (host) immune system and to activate the host immune system or to be recognized by a subject's epithelial cell, such as intestinal epithelial cell.

Cell envelope components are at times denoted in the art as pathogen-associated molecular patterns (PAMPs), which are molecules shared by and exclusive to microbes that are being recognized by host immune cell receptors called pattern recognition receptors (PRRs).

The cell envelope component may be or comprise at least one of a lipoprotein, a lipoglycan, a peptidoglycan, a lipopolysaccharide, a polysaccharide, a flagellin, a protein, a peptide, and a sugar residue.

The cell envelope component may be or comprise a peptidoglycan. The peptidoglycan may be a pseudopeptidoglycan. Pseudopeptidoglycan (also known as pseudomurein) is a major cell wall component of several archaea. In The pseudopeptidoglycan may be N-acetylglucosamine, N-acetyltalosaminuronic acid or any combination thereof.

The cell envelope component may be or comprise a polysaccharide. The polysaccharide may be an O-Antigen or a glucan. O-Antigens (also known as O-specific polysaccharides or O-side chains) are major component of the surface lipopolysaccharide (LPS) of Gram-negative bacteria and are highly variable in structure.A glucan is a polysaccharide derived from D-glucose, linked by glycosidic bonds. Glucans include alpha- glucans and beta-glucans and numbers clarify the type of O-glycosidic bond.

The cell envelope component may be a biopolymer comprising a galactose and arabinose residues. The biopolymer may be an arabinogalactan.

The cell envelope component may be or comprise a lipid residue. The lipid residue may be or comprise an archaeal lipid or a lipid A. Archaea lipids are characterized by comprising ether-linked lipids based on 2,3-dialkyl-sn-glycerol backbones. Non-limiting examples of archaea lipids include archaeol and caldarchaeol

The cell envelope component may be or comprise at least one of a lipoprotein, a lipoglycan and a lipopolysaccharide. The cell envelope component may be a lipopolysaccharide comprising lipid A.

The cell envelope component may be or comprise at least one of a pseudopeptidoglycans, archaeal lipids, an O-antigens, lipid A, arabinogalactans and beta glucan

The presence of a cell envelope component as detailed herein can be determined by any method known in the art, for example by using computational tools to identify genes that are encoding for the synthesis of specific cell envelope components detailed herein. In addition, such cell envelope component can be determined by any known method in the art.

The microbial consortium may be capable of activating at least one TLR.

The TLR family plays a fundamental role in pathogen recognition and activation of innate immunity. TLRs are highly conserved from Drosophila to humans and share structural and functional similarities. They recognize pathogen-associated molecular patterns (PAMPs) that are expressed on infectious agents, and mediate the production of cytokines necessary for the development of effective immunity. The various TLRs exhibit different patterns of expression.

The TLR may be selected from the group consisting of TLR1, TLR2, TLR3, TLR4, TLR5, TRL6, TLR7, TLR8, TLR9, and any combination thereof.

The microbial consortium may comprise at least one microorganism, for example the at least one microorganism being present at low-abundance in a microbiome of a reference human subject, may be capable of modulating at least one of TLR1, TLR2, TLR3, TLR4, TLR5, TRL6, TLR7, TLR8, TLR9 and any combination thereof.

The microbial consortium may comprise at least one microorganism, for example the at least one microorganism being present at low-abundance in a microbiome of a reference human subject, may be capable of modulating at least one of TLR2, TLR4, TLR5, TRL6 and any combination thereof.

The microbial consortium may comprise at least one microorganism, for example the at least one microorganism being present at low-abundance in a microbiome of a reference human subject, may be capable of modulating at least one of TLR2, TLR4 and any combination thereof.

The microbial consortium may be capable of activating at least one immune cell such as a T cell, a B cell, a NK cells Antigen presenting cell (APCs) such as macrophages or dendritic cells or a surface component of an immune cell.

The surface component of an immune cell may comprise at least one cluster of differentiation (CD).

The term cluster of differentiation (also known as cluster of designation or classification determinant) is a nomenclature protocol used for the identification and investigation of cell surface molecules providing targets for immunophenotyping of cells. CD molecules often act as receptors or ligands important to the cell. The at least one cluster of differentiation may be at least one of CD4, CD8, CD40, CD68, CD80, CD86, CD197, CD45, CD11b, CD103 and any combination thereof.

The microbial consortium may comprise at least one microorganism, for example the at least one microorganism being present at low-abundance in a microbiome of a reference human subject, may be capable of modulating the expression of at least one of CD4, CD8, CD40, CD68, CD80, CD86, CD197, CD45, CD11b, CD103 and any combination thereof.

The microbial consortium may comprise at least one microorganism, for example the at least one microorganism being present at low-abundance in a microbiome of a reference human subject, may be capable of modulating the expression of at least one of CD8 and CD68 and any combination thereof.

The microbial consortium may be capable of activating NF-κB. NF-κB is a protein complex that controls transcription of DNA, cytokine production and cell survival and plays a key role in regulating the immune response to infection.

The microbial consortium may be capable of activating a dendritic cell. Dendritic cells (DCs) are antigen-presenting cells of the immune system and their main function is to process antigen material and present it on the cell surface to the T cells of the immune system. Activated DCs migrate to the lymph nodes to interact with T cells and B cells to initiate and shape the adaptive immune response.

The microbial consortium may be capable of activating an inflammasome. An inflammasome is a multiprotein oligomer responsible for the activation of inflammatory responses and promotes the maturation and secretion of pro-inflammatory cytokines, such as Interleukin 1β (IL-1β) and Interleukin 18 (IL-18)

The microbial consortium may be capable of modulating an pro-inflammatory cytokine.

A pro-inflammatory cytokine (also denoted as an inflammatory cytokine) is a type of signaling molecule (a cytokine) that is secreted from immune cells like helper T cells (Th) and macrophages, and certain other cell types that promote inflammation.

Non-limiting examples of pro-inflammatory cytokines include interleukin-1 (IL-1), IL-12, IL-18, tumor necrosis factor alpha (TNF-α), interferon gamma (IFNγ), and granulocyte-macrophage colony stimulating factor (GM-CSF).

The pro-inflammatory cytokine may comprise at least one of interleukin-1 (IL-1), IL-2, IL-1β, IL-6, IL-12, IL-18, IL-22, IL-23 tumor necrosis factor alpha (TNF-α), interferon gamma (IFNγ), and granulocyte-macrophage colony stimulating factor (GM-CSF).

The pro-inflammatory cytokine may comprise at least one of at least one of IL-1β, IL-6, IL-12, TNF-α, IFNγ and any combination thereof.

The microbial consortium may comprises least one microorganism, for example the at least one microorganism being present at low-abundance in a microbiome of a reference human subject, may be capable of secreting at least one of IL-1, IL-1β, IL-6, IL-12, IL-18, TNF-α, IFNγ, GM-CSF and any combination thereof.

The microbial consortium may comprise at least one microorganism, for example the at least one microorganism being present at low-abundance in a microbiome of a reference human subject, may be capable of secreting at least one of IL-1β, IL-6, IL-12, TNF-α and any combination thereof.

The microbial consortium may be capable of activating secretion of tumor necrosis factor alpha (TNFα). TNFα is a cell signaling protein (cytokine) involved in systemic inflammation and is one of the cytokines that make up the acute phase reaction. The primary role of TNF is in the regulation of immune cells.

The microbial consortium may be capable of activating secretion of interferon gamma (IFNγ). IFNγ is a dimerized soluble cytokine that is critical for innate and adaptive immunity against viral, some bacterial and protozoal infections. It is an important activator of macrophages and inducer of Class II major histocompatibility complex (MHC) molecule expression.

The microbial consortium may be capable of activating mucin degradation. Activating mucin degradation in the context of the present disclosure refers to functioning to degrade host mucins. Host mucins are consisting predominantly of core 1-4 mucin-type O-glycans containing α- and β- linked N-acetyl-galactosamine, galactose and N-acetyl-glucosamine. These core structures are further elongated and frequently modified by fucose and sialic acid sugar residues via α1,2/3/4 and α2,3/6 linkages, respectively.

As described herein, the microorganisms that is present in low abundance were shown in the examples to modulate a variety of process.

The at least one microorganism may be present at low abundance in a reference human subject and may be capable of modulating at least one of acetic acid, propionic acid and butyric acid.

The at least one microorganism may be present at low abundance in a reference human subject comprises at least one of pseudopeptidoglycans, archaeal lipids, an O-antigens, lipid A, arabinogalactans and beta glucan.

The at least one microorganism may be present at low abundance in a reference human subject and may be capable of at least one of (i) modulating at least one of acetic acid, propionic acid and butyric acid and (ii) comprises at least one of pseudopeptidoglycans, archaeal lipids, an O-antigens, lipid A, arabinogalactans and beta glucan.

The at least one microorganism may be present at low abundance in a reference human subject and may be capable of modulating at least one of TLR2, TLR4, TLR5,TLR6, TLR7 and TLR8.

The at least one microorganism may be present at low abundance in a reference human subject and may be capable of at least one of (i) modulating at least one of acetic acid, propionic acid butyric acid, TLR2, TLR4, TLR5,TLR6, TLR7 and TLR8 and (ii) comprises at least one of pseudopeptidoglycans, archaeal lipids, an O-antigens, lipid A, arabinogalactans and beta glucan.

The at least one microorganism may be present at low abundance in a reference human subject may be capable of modulating at least one of TLR2 and TLR4.

The at least one microorganism may be present at low abundance in a reference human subject may be capable of modulating at least one of CD8 and CD68.

The at least one microorganism may be present at low abundance in a reference human subject may be capable of modulating at least one of TLR2, TLR4, CD8 and CD68.

The at least one microorganism may be present at low abundance in a reference human subject may be capable of modulating a dendritic cell.

The at least one microorganism may be present at low abundance in a reference human subject and may be capable of modulating at least one of IL-1β, IL-6, IL-12, TNF-α, and IFNγ. The at least one microorganism present at low abundance in a reference human subject may be capable of modulating at least one of IL-1β, IL-6, IL-12, and TNF-α.

The at least one microorganism present at low abundance in a reference human subject may be capable of at least one of modulating TLR2, modulating TLR4, modulating TLR5, modulating TLR6, modulating TLR7, modulating TLR8, secreting of IL-1β, secreting of IL-6, secreting of IL-12, secreting of TNF-α, secreting IFNγ, expressing CD8 and expressing CD68.

The at least one microorganism present at low abundance in a reference human subject may be capable of at least one of modulating TLR2, modulating TLR4, secreting of IL-1β, secreting of IL-6, secreting of IL-12, secreting of TNF-α, secreting IFNγ, expressing CD8 or expressing CD68.

The at least one microorganism present at low abundance in a reference human subject may be capable of at least one of (i) modulating at least one of an acetic acid, propionic acid, butyric acid, TLR2, TLR4, TLR5, TLR6, TLR7, TLR8, CD8, CD68, IL-1β, IL-6, IL-12, TNF-α, and IFNγ, and (ii) comprising at least one of pseudopeptidoglycans, archaeal lipids, an O-antigens, lipid A, arabinogalactans and beta glucan.

The at least one microorganism present at low abundance in a reference human subject may be capable of at least one of (i) modulating at least one of an TLR2, TLR4, CD8, CD68, IL-1β, IL-6, IL-12, and TNF-α and (ii) comprising at least one of pseudopeptidoglycans, archaeal lipids, an O-antigens, lipid A, arabinogalactans and beta glucan.

As detailed above, the activation of the immune response is achieved by the unique identified microorganisms.

The microbial consortium may comprise at least one microorganism from the *Methanosphaera* genus, *Oenococcus* genus, *Cetobacterium* genus, *Corynebacterium* genus and *Clostridium* genus.

The microbial consortium may comprise at least one, at least two, at least three microorganism from the *Oenococcus* genus, *Cetobacterium* genus, *Corynebacterium* genus and *Clostridium* genus.

The at least one microorganism of low abundance may be at least one of the *Methanosphaera* genus, *Oenococcus* genus, *Cetobacterium* genus, *Corynebacterium* genus and *Clostridium* genus.

The at least one microorganism of low abundance may be at least one of the *Oenococcus* genus, *Cetobacterium* genus, *Corynebacterium* genus and *Clostridium* genus.

The microbial consortium may comprise at least one microorganism from the *Methanosphaera* genus. *Methanosphaera* genus may be denoted by Taxonomy ID (or taxid): 2316. *Methanosphaera* is a genus of microorganisms (archaea) within the family Methanobacteriaceae.

The microbial consortium may comprise at least one microorganism from the *Oenococcus* genus. *Oenococcus* genus may be denoted by Taxonomy ID: 46254. *Oenococcus* is a genus of gram-positive bacteria, within the family Leuconostocaceae. Non-limiting examples of *Oenococcus* genus include *Oenococcus oeni* and *Oenococcus kitaharae.*

The microbial consortium may comprise at least one microorganism from the *Cetobacterium* genus. Cetobacterium genus may be denoted by Taxonomy ID: 180162. *Cetobacterium* is a genus of Gram-negative bacteria from the family of *Fusobacteriaceae.*

The microbial consortium may comprise at least one microorganism species (type) from the *Corynebacterium* genus. *Corynebacterium* genus may be denoted by Taxonomy ID: 1716. *Corynebacterium* is a genus of bacteria that are Gram-positive and aerobic and are bacilli (rod-shaped).

The microbial consortium may comprise at least one microorganism from the *Clostridium* genus. *Clostridium* genus may be denoted by Taxonomy ID: 1485. *Clostridium* is a genus of Gram-positive bacteria.

The microbial consortium may comprise at least one microorganism from the *Methanosphaera stadtmanae* species, *Oenococcus oeni* species, *Clostridium cellulovorans* species, *Cetobacterium somerae* species, *Corynebacterium glyciniphilum* species and *Clostridium tyrobutyricum* species.

The microbial consortium may comprise at least one, at least two, at least three microorganism from the *Oenococcus oeni* species, *Clostridium cellulovorans* species, *Cetobacterium somerae* species, *Corynebacterium glyciniphilum* species and *Clostridium tyrobutyricum* species.

The at least one microorganism of low abundance may be at least one from the *Methanosphaera stadtmanae* species, *Oenococcus oeni* species, *Clostridium cellulovorans* species, *Cetobacterium somerae* species, *Corynebacterium glyciniphilum* species and *Clostridium tyrobutyricum* species.

The at least one microorganism of low abundance may be at least one from the *Oenococcus oeni* species, *Clostridium cellulovorans* species, *Cetobacterium somerae* species, *Corynebacterium glyciniphilum* species and *Clostridium tyrobutyricum* species.

The at least one microorganism of low abundance may be at least one from the *Oenococcus oeni* species, *Cetobacterium somerae* species, *Corynebacterium glyciniphilum* species and *Clostridium tyrobutyricum* species.

The at least one microorganism of low abundance may be at least one from the *Clostridium cellulovorans* species, *Cetobacterium somerae* species, and *Clostridium tyrobutyricum* species.

The at least one microorganism of low abundance may be at least one from the *Clostridium cellulovorans* species and *Cetobacterium somerae* species.

The microbial consortium may comprise at least one microorganism from the *Methanosphaera stadtmanae* species. *Methanosphaera stadtmanae* species may be denoted by Taxonomy ID: 2317.

The microbial consortium may comprise at least one microorganism from the *Oenococcus oeni* species. *Oenococcus oeni* species may be denoted by Taxonomy ID: 1247.

The microbial consortium may comprise at least one microorganism from the *Cetobacterium somerae* species. *Cetobacterium somerae* species may be denoted by Taxonomy ID: 188913.

The microbial consortium may comprise at least one microorganism from the *Corynebacterium glyciniphilum* species. *Corynebacterium glyciniphilum* species may be denoted by Taxonomy ID: 1404244.

The microbial consortium may comprise at least one microorganism from the *Clostridium tyrobutyricum* species. *Clostridium tyrobutyricum* species may be denoted by Taxonomy ID: 1519.

The microbial consortium may comprise at least one microorganism from the *Clostridium cellulovorans* species. *Clostridium cellulovorans* species may be denoted by Taxonomy ID: 1493.

In the context of the present disclosure, identification of microorganism from a biological sample of a human subject may be done using any conventional method in the microbiology field. For example and without being limited thereto identification of bacteria from a biological sample of a human subject may be done using 16S rRNA (ribosomal RNA) sequencing. Identification of isolated microorganism may be done by conducting similarity analysis between the 16S rRNA gene of the isolated microorganism to different microorganism's 16S rRNA gene sequences available in database. This analysis may be done in order to explore the similarity between a given sequence and all of the available sequences in a database and obtaining the best matched sequences by calculation of a score for the examined similarity. Identity analysis may be conducted by any appropriate program, for example Basic Local Alignment Search Tool (BLAST^{®}) using publicly available databases, for example National Center for Biotechnology Information (NCBI).

At least one of the two or more microorganisms may comprise 16S rRNA sequences having between 85% to 99%, at times between 90% to 99%, at times between 95% to 99%, at times between 96% to 99%, at times between 97% to 99%, at times between 98% to 99% identity with at least one nucleic acid sequences selected from SEQ ID NO:1, SEQ ID NO:2, SEQ ID NO:3, SEQ ID NO:4, SEQ ID NO:5 and SEQ ID NO:6.

At least one of the two or more microorganisms may comprise 16S rRNA sequences having at least at least 85%, at least 90%, at least 95%, at least 96%, at least 97%, at least 98%, at least 99%, at least 99.5% identity with at least one nucleic acid sequences selected from SEQ ID NO:1, SEQ ID NO:2, SEQ ID NO:3, SEQ ID NO:4, SEQ ID NO:5 and SEQ ID NO:6. It should be noted that each one of the % identity constitutes a separate disclosure.

The threshold sequence identity may be 85%, at times 86%, at times 87%, at times 88%, at times 89%, at times 90%, at times 91%, at times 92%, at times 93%, at times 94%, at times 95%, at times 96%, at times 97%, at times 98%, at times 99%, at times 99.5% with each one of the % identity denoted herein constitute a separate disclosure
The term identity (% identity) as used herein refer to two or more nucleic acid sequences, that are the same. In the context of the present disclosure the sequence identity encompasses transcription changes of DNA to RNA, e.g. T and U are considered identical. The identity may exist over a region of a sequence that is considered by those versed in the art as the variable region of the 16S rRNA. The identity may exist over the length the 16S rRNA or a portion thereof of the variable region.

The % identity between two or more nucleic acid sequences is determined for the two or more sequences when compared and aligned for maximum correspondence. In the context of the present disclosure, sequences (nucleic acid) as described herein having % identity are considered to have the same function/activity of the original sequence to which identity is calculated to.

The microbial consortium may comprise at least *Oenococcus oeni* PSU-1. *Oenococcus oeni* PSU-1 may be denoted by Taxonomy ID: 203123. The 16S rRNA sequence of *Oenococcus oeni* PSU-1 is provided by SEQ ID NO:1.

The microbial consortium may comprises at least *Clostridium tyrobutyricum* KCTC *5387. Clostridium tyrobutyricum* KCTC 5387 may be denoted by Taxonomy ID: 1121342. The 16S rRNA sequence of *Clostridium tyrobutyricum* KCTC 5387 is provided by GenBank accession No. NR_044718.2 (SEQ ID NO:2).

The microbial consortium may comprise at least *Cetobacterium somerae* ATCC BAA-474. *Cetobacterium somerae* ATCC BAA-474 may be denoted by Taxonomy ID: 1319815. The 16S rRNA sequence of *Cetobacterium somerae* ATCC BAA-474 is provided by GenBank accession No. AJ438155.2 (SEQ ID NO:3).

The microbial consortium may comprise at least *Corynebacterium glyciniphilum* AJ 3170. *Corynebacterium glyciniphilum* AJ 3170 may be denoted by Taxonomy ID: 1404245. The 16S rRNA sequence of *Corynebacterium glyciniphilum* AJ 3170 is provided by GenBank accession No. NR_121782.1 (SEQ ID NO:4).

The microbial consortium may comprise at least *Clostridium cellulovorans 743B. Clostridium cellulovorans 743B* may be dented by Taxonomy ID: 573061. The 16S rRNA sequence of *Clostridium cellulovorans 743B* is provided by GenBank accession No. NR_119029.1 (SEQ ID NO:5).

The microbial consortium may comprise at least *Methanosphaera stadtmanae* DSM3091. *Methanosphaera stadtmanae* DSM3091 may be denoted by Taxonomy ID: 339860. The 16S rRNA sequence *of Methanosphaera stadtmanae* DSM3091 is provided by GenBank accession No. NR_028236.1 (SEQ ID NO:6).

The microbial consortium may comprise two microorganisms, as identified above. The microbial consortium may comprise a combination of two, three, four, five or more microorganisms as identified above.

At least one of the two or more microorganisms in the microbial consortium may, for example be of low abundance in the microbiome of a reference subject, comprises 16S rRNA sequence of at least one of SEQ ID NO:1, SEQ ID NO:2, SEQ ID NO:3, SEQ ID NO:4, SEQ ID NO:5 and SEQ ID NO:6.

The two or more microorganisms in the microbial consortium may, for example be of low abundance in the microbiome of a reference subject, comprises 16S rRNA sequence of at least two of SEQ ID NO:1, SEQ ID NO:2, SEQ ID NO:3, SEQ ID NO:4, SEQ ID NO:5 and SEQ ID NO:6.

The microbial consortium may comprise at least one microorganism, at least two, at least three, at least four and at times five of low abundance having a 16S rRNA sequences selected from the group consisting of SEQ ID NO:1, SEQ ID NO:2, SEQ ID NO:3, SEQ ID NO:4 and SEQ ID NO:5.

The microbial consortium may comprise in some embodiments at least one, at least two, at least three, or at least four microorganisms having a 16S rRNA sequence as denoted by SEQ ID NO:1, SEQ ID NO:2, SEQ ID NO:3, SEQ ID NO:4 and SEQ ID NO:5.

The microbial consortium may comprise in some embodiments one, two, three, four, or five microorganisms having a 16S rRNA sequence as denoted by SEQ ID NO:1, SEQ ID NO:2, SEQ ID NO:3, SEQ ID NO:4 and SEQ ID NO:5.

The microbial consortium may comprise at least one, at least two or more microorganisms selected *from Methanosphaera stadtmanae* DSM3091, *Oenococcus oeni* PSU-1, *Clostridium tyrobutyricum* KCTC 5387, *Cetobacterium somerae* ATCC BAA-*474, Corynebacterium glyciniphilum* AJ 3170, and *Clostridium cellulovorans 743B.*

The microbial consortium may comprise two or more microorganisms selected from *Methanosphaera stadtmanae* DSM3091, *Oenococcus oeni* PSU-1, *Clostridium tyrobutyricum* KCTC 5387, *Cetobacterium somerae* ATCC BAA-474.

The microbial consortium may comprise one, two, three, four or five microorganisms selected from *Oenococcus oeni* PSU-1, *Clostridium tyrobutyricum* KCTC 5387, *Cetobacterium somerae* ATCC BAA-474, *Corynebacterium glyciniphilum* AJ 3170, and *Clostridium cellulovorans 743B.*

The microbial consortium may comprises one, two, three or four microorganisms selected from *Oenococcus oeni* PSU-1, *Clostridium tyrobutyricum* KCTC 5387, *Cetobacterium somerae* ATCC BAA-474 and *Corynebacterium glyciniphilum* AJ 3170.

The microbial consortium may comprises one, two or three microorganisms selected from *Cetobacterium somerae* ATCC BAA-474, *Clostridium tyrobutyricum* KCTC 5387, and *Clostridium cellulovorans 743B.*

The microbial consortium may comprise one or two microorganisms selected from *Cetobacterium somerae* ATCC BAA-474 and *Clostridium tyrobutyricum* KCTC 5387.

The microbial consortium may comprise also at least one high-abundance microorganism from a microbiome of a human reference subject.

The high-abundance microorganism may be the same microorganism as the microorganism secreting at least one of (i) capable of at least one SCFA, (ii) capable of modulating lactate and (iii) comprises at least one cell envelope component.

The high-abundance microorganism may be a different microorganism than the microorganism secreting at least one of (i) capable of at least one SCFA, (ii) capable of modulating lactate and (iii) comprise at least one cell envelope component.

The high-abundance microorganism may be a different microorganism than the microorganism modulating (producing and/or secreting) a short chain fatty acid (SCFA).

The high-abundance microorganism may be a different microorganism than the microorganism modulating (producing and/or secreting) lactate.

The high-abundance microorganism may be a different microorganism than the microorganism comprising at least one cell envelope component.

Thus, the microbial consortium may comprise two or more microorganisms, at least one of the two or more microorganisms may be present at low-abundance in a microbiome of a reference human subject and at least one other of the two or more microorganisms may be present at high-abundance in a microbiome of a reference human subject and having at least one of (i) capable of at least one SCFA, (ii) capable of modulating lactate and (iii) comprises at least one cell envelope component.

The microbial consortium may comprise three or more microorganisms, at least one of the three or more microorganisms may be present at low-abundance in a microbiome of a reference human subject, at least one other of the three or more microorganisms secretes at least one of having at least one of (i) capable of at least one SCFA, (ii) capable of modulating lactate and (iii) comprises at least one cell envelope component or a different bacteria and at least one of the three or more microorganisms may be present at high-abundance in a microbiome of a reference human subject.

The term *high-abundance* as used herein refer to a microorganism that is present in the microbiome of a reference subject in an amount that is above a predetermined standard value/s or cutoff value/s. The predetermined standard value/s or cutoff value/s may be determined as an average value for a large, heterogonous cohort of subjects or alternatively be determined for a specific population of reference subjects. The reference human subject may be a healthy subject. A healthy subject is a subject that is not diagnosed with a disease to be treated using the microbial consortium. The reference human subject may be a subject diagnosed with at least one disease, optionally a disease that can be treated using the microbial consortium. A high abundance microorganism may have an average relative abundance of more than 0.5%, at times more than 0.6%, at times more than 0.7%, at times more than 0.8%, at times even more than 0.9%, at times even more than 1.5%, at times even more than 2%.A high abundance microorganism may have an average relative abundance of between about 0.5% to about 5%, at times between about 0.6% to about 5%, at times between about 0.7% to about 5%, at times between about 1% to about 5%, at times between about 1.5% to about 5% at times between about 2% to about 5%.

The high abundance microorganism may carry a cell envelope component that is recognized by a subject's immune system. he high abundance microorganism may carry a cell envelope component that activates a subject's immune system as described herein.

The high-abundance microorganism may comprise at least one microorganism from the *Bacteroides* genus, *Ruminococcus* genus, *Lactobacillus* genus, *Veillonella* genus, *Bifidobacterium* genus, *Alistipes* genus and *Akkermansia* genus.

The high-abundance microorganism may comprise at least one microorganism from the *Bacteroides* genus, *Lactobacillus* genus, or *Veillonella* genus.

The high-abundance microorganism may comprise at least one microorganism from the *Bacteroides* genus. *Bacteroides* genus may be dented by Taxonomy ID: 816. *Bacteroides* is a genus of Gram-negative, obligate anaerobic bacteria.

The high-abundance microorganism may comprise at least one microorganism from the *Ruminococcus* genus. *Ruminococcus* genus may be denoted by Taxonomy ID: 1263. *Ruminococcus* is a genus of bacteria in the class Clostridia that are anaerobic, Gram-positive microbes.

The high-abundance microorganism may comprise at least one microorganism species or stain from the *Lactobacillus* genus. *Lactobacillus* genus may be dented by Taxonomy ID: 1578. *Lactobacillus* is a genus of Gram-positive, facultative anaerobic or microaerophilic, rod-shaped, non-spore-forming bacteria that are a major part of the lactic acid bacteria group (i.e., they convert sugars to lactic acid).

The high-abundance microorganism may comprise at least one microorganism species or strain from the *Veillonella* genus. *Veillonella* genus may be dented by Taxonomy ID: 29465. *Veillonella* is a genus of Gram-negative bacteria anaerobic cocci, known for its lactate fermenting abilities.

The high-abundance microorganism may comprise at least one microorganism species or strain from the *Bifidobacterium* genus. *Bifidobacterium* genus may be dented by Taxonomy ID: 1678. *Bifidobacterium* is a genus of gram-positive, nonmotile, often branched anaerobic bacteria.

The high-abundance microorganism may comprise at least one microorganism species or strain from the *Alistipes* genus. *Alistipes* genus may be dented by Taxonomy *ID: 239759.Alistipes* is a genus in the phylum Bacteroidetes.

The high-abundance microorganism may comprise at least one microorganism species or strain from the *Akkermansia* genus. *Akkermansia* genus may be dented by Taxonomy ID: 239934 . *Akkermansia* is a genus in the phylum Verrucomicrobia.

The microbial consortium may comprise at least one microorganism from the *Bacteroides caccae* species, *Ruminococcus albus* species, *Lactobacillus casei* species, *Veillonella parvula* species, *Alistipes putredinis* species, *Bifidobacterium breve* species and *Akkermansia muciniphila* species.

The microbial consortium may comprise at least one microorganism, at least two microorganisms, at least three microorganisms from the *Bacteroides caccae* species, *Lactobacillus casei* species or *Veillonella parvula* species.

The microbial consortium may comprise at least one microorganism, at least two microorganisms from the *Bacteroides caccae* species, or *Veillonella parvula* species.

The microbial consortium may comprise at least one microorganism from the *Ruminococcus albus* species. *Ruminococcus albus* species may be denoted by Taxonomy ID: 1264.

The microbial consortium may comprise at least one microorganism from the *Lactobacillus casei* species. *Lactobacillus casei* species may be denoted by Taxonomy ID: 1582.

The microbial consortium may comprise at least one microorganism from the *Veillonella parvula* species. *Veillonella parvula* species may be denoted by Taxonomy ID: 29466.

The microbial consortium may comprise at least one microorganism from the *Alistipes putredinis* species. *Alistipes putredinis* species may be denoted by Taxonomy ID: 28117.

The microbial consortium may comprise at least one microorganism from the *Bifidobacterium breve* species. *Bifidobacterium breve* species may be denoted by Taxonomy ID: 1685.

The microbial consortium may comprise at least one microorganism from the *Akkermansia muciniphila* species. *Akkermansia muciniphila* species may be denoted by Taxonomy ID: 239935.

The microbial consortium may comprise at least one microorganism from the *Bacteroides caccae* species. *Bacteroides caccae* species may be denoted by Taxonomy ID: 47678.

At least one of the two or more microorganisms may comprise 16S rRNA sequences having between 85% to 99%, at times between 90% to 99%, at times between 95% to 99%, at times between 96% to 99%, at times between 97% to 99%, at times between 98% to 99% identity with at least one nucleic acid denoted by SEQ ID NO:7, SEQ ID NO:8, SEQ ID NO:9, SEQ ID NO:10, SEQ ID NO:11, SEQ ID NO:12, SEQ ID NO:13 and SEQ ID NO:14, at times with at least one nucleic acid denoted by SEQ ID NO:7, SEQ ID NO:8 and SEQ ID NO:9.

At least one of the two or more microorganisms may comprise 16S rRNA sequences having at least 85%, at least 90%, at least 95%, at least 96%, at least 97%, at least 98%, at least 99% identity with at least one nucleic acid denoted by SEQ ID NO:7, SEQ ID NO:8, SEQ ID NO:9, SEQ ID NO:10, SEQ ID NO:11, SEQ ID NO:12, SEQ ID NO:13 and SEQ ID NO:14, at times with at least one nucleic acid denoted by SEQ ID NO:7, SEQ ID NO:8 and SEQ ID NO:9. It should be noted that each one of the % identity constitutes a separate disclosure.

As noted herein, the threshold sequence identity may be 85%, at times 86%, at times 87%, at times 88%, at times 89%, at times 90%, at times 91%, at times 92%, at times 93%, at times 94%, at times 95%, at times 96%, at times 97%, at times 98%, at times 99% with each one of the % identity denoted herein constitute a separate disclosure
The high-abundance microorganism may comprise at least one of *Bacteroides caccae* CL03T12C61, *Ruminococcus albus 7, Lactobacillus casei ATCC27139, Veillonella parvula ATCC 17745, Bifidobacterium breve* ATCC 15700, *Alistipes putredinis* DSM 17216, *Akkermenia muciniphila* ATCC BAA-835 and *Akkermansia muciniphila* YL44.

The high-abundance microorganism may comprise at least one, at least two of *Bacteroides caccae* CL03T12C61, *Lactobacillus casei ATCC27139* or *Veillonella parvula ATCC 17745.*

The high-abundance microorganism may comprise one, two or three *of Bacteroides caccae* CL03T12C61, *Lactobacillus casei ATCC27139* or *Veillonella parvula ATCC 17745.*

The high-abundance microorganism may comprise one, two or three *of Bacteroides caccae* CL03T12C61 or *Veillonella parvula ATCC 17745.*

The high-abundance microorganism may comprise one, two or three of *Lactobacillus casei ATCC27139* or *Veillonella parvula ATCC 17745.*

The high-abundance microorganism may comprise one, two or three of *Lactobacillus casei ATCC27139* or *Bacteroides caccae* CL03T12C61.

The high-abundance microorganism may comprise *Veillonella parvula ATCC 17745. Veillonella parvula ATCC* 17745 may be denoted by Taxonomy ID: 686660. The 16S rRNA sequence of *Veillonella parvula ATCC 17745* is provided by Gene Bank accession No. AY995769.1 (SEQ ID NO:7).

The high-abundance microorganism may comprise *Bacteroides caccae* CL03T12C61. *Bacteroides caccae* CL03T12C61 may be denoted by Taxonomy ID: 997873. The 16S rRNA sequence of *Bacteroides caccae* CL03T12C61 is provided by SEQ ID NO:8.

The high-abundance microorganism may comprise *Lactobacillus casei ATCC27139.* The 16S rRNA sequence of *Lactobacillus casei ATCC27139* is provided by Gene Bank accession No. AB531131.1 (SEQ ID NO:9)
The high-abundance microorganism may comprise *Ruminococcus albus 7. Ruminococcus albus* 7 may be denoted by Taxonomy ID: 697329. The 16S rRNA sequence of *Ruminococcus albus* 7 is provided by Gene Bank accession No. NR_025929.1 (SEQ ID NO:10).

The high-abundance microorganism may comprise *Bifidobacterium breve* ATCC *15700. Bifidobacterium breve* ATCC 15700 may be denoted by Taxonomy ID: 518634. The 16S rRNA sequence of *Veillonella parvula ATCC 17745* is provided by Gene Bank accession No. NR_040783.1 (SEQ ID NO:11).

The high-abundance microorganism may comprise *Alistipes putredinis* DSM *17216. Alistipes putredinis* DSM 17216 may be denoted by Taxonomy ID: 445970. The 16S rRNA sequence *of Alistipes putredinis* DSM 17216 is provided Gene Bank accession No. NR_025909.1 (SEQ ID NO:12)
The high-abundance microorganism may comprise *Akkermansia muciniphila* ATCC BAA-835. *Akkermansia muciniphila* ATCC BAA-835 may be denoted by Taxonomy ID: 349741. The 16S rRNA sequence of *Akkermansia muciniphila* ATCC BAA-835 is provided by Gene Bank accession No. NR_042817.1 (SEQ ID NO:13).

The high-abundance microorganism may comprise *Akkermansia muciniphila* YL44. The 16S rRNA sequence of *Akkermansia muciniphila* YL44 is provided by SEQ ID NO:14.

Also provided is a microbial consortium comprising two or more purified or isolated microorganisms, at least one of the two or more microorganisms may be present at low abundance in a reference human subject being capable of at least one of (i) modulating at least one of an acetic acid, propionic acid, butyric acid, TLR2, TLR4, TLR5, TLR6, TLR7, TLR8, CD8, CD68, IL-1β, IL-6, IL-12, and TNF-α and IFNγ and (ii) comprises at least one of pseudopeptidoglycans, archaeal lipids, an O-antigens, lipid A, arabinogalactans and beta glucan and at least one different microorganism of the two or more microorganisms carries a cell envelope component that is recognized by a subject's immune system. The at least one different microorganism may be a microorganism being present at low abundance in a reference human subject or a microorganism being present at high abundance in a reference human subject.

Also provided is a microbial consortium comprising two or more purified or isolated microorganisms, at least one of the two or more microorganisms may be present at low abundance in a reference human subject being capable of at least one of (i) modulating at least one of TLR2, TLR4, TLR5, TLR6, TLR7, TLR8, CD8, CD68, IL-1β, IL-6, IL-12, TNF-α and IFNγ and (ii) comprises at least one of pseudopeptidoglycans, archaeal lipids, an O-antigens, lipid A, arabinogalactans and beta glucan and at least one different microorganism of the two or more microorganisms comprises at least one of pseudopeptidoglycans, archaeal lipids, an O-antigens, lipid A, arabinogalactans and beta glucan. The at least one different microorganism may be a microorganism being present at low abundance in a reference human subject or a microorganism being present at high abundance in a reference human subject.

Also provided is a microbial consortium comprising two or more purified or isolated microorganisms, at least one microorganism present at low abundance in a reference human subject being capable of at least one of (i) modulating at least one of TLR2, TLR4, CD8, CD68, IL-1β, IL-6, IL-12, TNF-α, and IFNγ and (ii) comprises at least one of pseudopeptidoglycans, archaeal lipids, an O-antigens, lipid A, arabinogalactans and beta glucan and at least one different microorganism that carries a cell envelope component that is recognized by a subject's immune system. The at least one different microorganism may be a microorganism being present at low abundance in a reference human subject or a microorganism being present at high abundance in a reference human subject.

Also provided is a microbial consortium comprising two or more purified or isolated microorganisms, comprises at least one microorganism present at low abundance in a reference human subject being capable of at least one of (i) modulating at least one of TLR2, TLR4, CD8, CD68, IL-1β, IL-6, IL-12 TNF-α, and IFNγ and (ii) comprises at least one of pseudopeptidoglycans, archaeal lipids, an O-antigens, lipid A, arabinogalactans and beta and at least one different microorganism that comprises at least one of pseudopeptidoglycans, archaeal lipids, an O-antigens, lipid A, arabinogalactans and beta glucan. The at least one different microorganism may be a microorganism being present at low abundance in a reference human subject or a microorganism being present at high abundance in a reference human subject.

Also provided is a microbial consortium comprising two or more purified or isolated microorganisms present at low abundance in a reference human subject being capable of modulating at least one of TLR2, TLR4, CD8, CD68, IL-1β, IL-6, IL-12 TNF-α and IFNγ.

Also provided is a microbial consortium comprising two or more purified or isolated microorganisms present at low abundance in a reference human subject being capable of modulating at least one of TLR2, TLR4, CD8, CD68, IL-1β, IL-6, IL-12 and TNF-α.

Also provided is a microbial consortium comprising two or more purified or isolated microorganisms, at least one of the two or more microorganisms having a 16S rDNA sequence that is at least 95%, at least 96%, at least 97%, at least 98%, at least 99%, at least 99.5% identical to at least one16S rDNA sequence denoted by of SEQ ID NO: 1, SEQ ID NO:2, SEQ ID NO:3, SEQ ID NO:4 and SEQ ID NO:5 and at least one different microorganism of the two or more microorganisms comprises at least one of pseudopeptidoglycans, archaeal lipids, an O-antigens, lipid A, arabinogalactans and beta glucan. The at least one different microorganism may be a microorganism being present at low abundance in a reference human subject or a microorganism being present at high abundance in a reference human subject.

Also provided is a microbial consortium comprising two or more purified or isolated microorganisms, at least one of the two or more microorganisms may be present at low abundance in a reference human subject being capable of modulating at least one of TLR2, TLR4, TLR5, TLR6, TLR7, TLR8, CD8, CD68, IL-1β, IL-6, IL-12, TNF-α and IFNγ and at least one different microorganism of the two or more microorganisms having a 16S rDNA sequence that is at least 95%, at least 96%, at least 97%, at least 98%, at least 99%, at least 99.5% identical to at least one 16S rDNA sequence denoted by of SEQ ID NO:6, SEQ ID NO:7 and SEQ ID NO:8. The at least one different microorganism may be a microorganism being present at low abundance in a reference human subject or a microorganism being present at high abundance in a reference human subject.

Also provided is a microbial consortium comprising two or more purified or isolated microorganisms, at least one of the two or more microorganisms may be present at low abundance in a reference human subject being capable of modulating at least one of TLR2, TLR4, CD8, CD68, IL-1β, IL-6, IL-12 and TNF-α and at least one different microorganism of the two or more microorganisms having a 16S rDNA sequence that is at least 95%, at least 96%, at least 97%, at least 98%, at least 99%, at least 99.5% identical to at least one16S rDNA sequence denoted by of SEQ ID NO:6, SEQ ID NO:7 and SEQ ID NO:8.

Also provided is a microbial consortium comprising two or more purified or isolated microorganisms having a 16S rDNA sequence that is at least 95%, at least 96%, at least 97%, at least 98%, at least 99%, at least 99.5% identical to at least one, at least two, at least three, at least four 16S rDNA sequences listed in Table 1.

Also provided is a microbial consortium comprising two or more purified or isolated microorganisms having a 16S rDNA sequence that is at least 95%, at least 96%, at least 97%, at least 98%, at least 99%, at least 99.5% identical to at least one, at least two, at least three, at least four16S rDNA sequence denoted by of SEQ ID NO:1, SEQ ID NO:2, SEQ ID NO:3, SEQ ID NO:4, SEQ ID NO:5, SEQ ID NO:6, SEQ ID NO:7 and SEQ ID NO:8.

Also provided is a microbial consortium comprising two or more purified or isolated microorganisms having a 16S rDNA sequence that is at least 95%, at least 96%, at least 97%, at least 98%, at least 99%, at least 99.5% identical to one, two, three, four, five, six seven, eight 16S rDNA sequence denoted by of SEQ ID NO:1, SEQ ID NO:2, SEQ ID NO:3, SEQ ID NO:4, SEQ ID NO:5, SEQ ID NO:6, SEQ ID NO:7 and SEQ ID NO:8.

The microbial consortium may comprise two or more microorganisms selected from *Methanosphaera stadtmanae* DSM3091, *Oenococcus oeni* PSU-1, *Clostridium tyrobutyricum* KCTC 5387, *Cetobacterium somerae* ATCC BAA-474, *Corynebacterium glyciniphilum* AJ 3170, *Bacteroides caccae* CL03T12C61, *Ruminococcus albus 7, Lactobacillus casei ATCC27139, Veillonella parvula ATCC 17745* and *Clostridium cellulovorans 743B.*

The microbial consortium may comprise three, at times four, at times five, at times six, at times seven, at times eight, at times nine, at times ten or more microorganisms selected from *Methanosphaera stadtmanae* DSM3091, *Oenococcus oeni* PSU-1, *Clostridium tyrobutyricum* KCTC 5387, *Cetobacterium somerae* ATCC BAA-474, *Corynebacterium glyciniphilum* AJ 3170, *Bacteroides caccae* CL03T12C61, *Ruminococcus albus 7, Lactobacillus casei ATCC27139, Veillonella parvula ATCC 17745, Clostridium cellulovorans 743B, Bifidobacterium breve* ATCC15700, *Alistipes putredini* DSM 17216, *Akkermansia muciniphila* ATCC BAA-835 and *Akkermansia muciniphila* YL44.

The microbial consortium may comprise at least two isolated or purified microorganisms belonging to the genus, spices or strain identified by NCBI Taxonomy IDs selected from the group consisting of NCBI Taxonomy ID: 2316, 46254, 180162, 1716, 1485, 2317, 1247, 188913, 1404244, 1519, 1493, 203123, 1121342, 1319815, 1404245, 573061, 339860, 816, 1263, 1578, 29465, 1678, 239759, 239934, 1264, 1582, 29466, 28117, 1685, 239935, 47678, 686660, 997873, 697329, 518634, 445970, 349741,

The microbial consortium may comprise two or more microorganisms selected from *Oenococcus oeni* PSU-1, *Methanosphaera stadtmanae* DSM3091, *Clostridium tyrobutyricum* KCTC 5387, *Cetobacterium somerae* ATCC BAA-474 and *Corynebacterium glyciniphilum* AJ 3170.

The microbial consortium may comprise two or more microorganisms are selected from *Oenococcus oeni* PSU-1, *Methanosphaera stadtmanae* DSM3091, *Clostridium tyrobutyricum* KCTC 5387, and *Cetobacterium somerae* ATCC BAA-474.

The microbial consortium may consists from *Oenococcus oeni* PSU-1, *Methanosphaera stadtmanae* DSM3091, *Clostridium tyrobutyricum* KCTC 5387, and *Cetobacterium somerae* ATCC BAA-474.

The microbial consortium may comprise two or more microorganisms are selected from *Oenococcus oeni* PSU-1, *Methanosphaera stadtmanae* DSM3091, *Ruminococcus albus 7, Bacteroides caccae CL03T12C61* and *Clostridium tyrobutyricum* KCTC 5387.

The microbial consortium may comprise two or more microorganisms are selected from *Oenococcus oeni* PSU-1, *Methanosphaera stadtmanae* DSM3091, *Ruminococcus albus* 7 and *Bacteroides caccae CL03T12C61.*

The microbial consortium may consist from *Oenococcus oeni* PSU-1, *Methanosphaera stadtmanae* DSM3091, *Ruminococcus albus* 7 and *Bacteroides caccae CL03T12C61.*

The microbial consortium may comprise two or more microorganisms are selected from *Methanosphaera stadtmanae* DSM3091, *Lactobacillus casei ATCC27139, Veillonella parvula ATCC 17745* and *Ruminococcus albus 7.*

The microbial consortium may consist from *Methanosphaera stadtmanae* DSM3091, *Lactobacillus casei ATCC27139, Veillonella parvula ATCC 17745* and *Ruminococcus albus 7.*

The microbial consortium may comprise at least one, at least two, at least three and at times, at least all of *Corynebacterium glyciniphilum* AJ 3170, *Cetobacterium somerae* ATCC BAA-474, *Clostridium tyrobutyricum* KCTC 5387 and *Oenococcus oeni* PSU-1.

The microbial consortium may comprise *Corynebacterium glyciniphilum* AJ 3170, *Cetobacterium somerae* ATCC BAA-474, *Clostridium tyrobutyricum* KCTC 5387 and *Oenococcus oeni* PSU-1. This microbial consortium comprising the four listed bacterial strains is referred herein as Consortium 1 ("Cons. 1")

The microbial consortium may comprise at least one, at least two, at least three and at times, at least all of *Bacteroides caccae CL031T12C61, Clostridium cellulovorans 743B, Lactobacillus casei ATCC27139, Veillonella parvula ATCC 17745.*

The microbial consortium may comprise *Bacteroides caccae CL03T12C61, Clostridium cellulovorans 743B, Lactobacillus casei ATCC27139, Veillonella parvula ATCC 17745.* This microbial consortium comprising the four listed bacterial strains is referred herein as Consortium 2 ("Cons. 2")

The microbial consortium may comprise at least one, at least two, at least three, at least four, at least five, at least six, at least seven and at times, at least all of *Corynebacterium glyciniphilum AJ 3170, Cetobacterium somerae ATCC BAA-474, Clostridium tyrobutyricum KCTC 5387, Oenococcus oeni PSU-1, Bacteroides caccae CL03T12C61, Clostridium cellulovorans 743B, Lactobacillus casei ATCC27139 and Veillonella parvula ATCC 17745.*

The microbial consortium may comprise *Corynebacterium glyciniphilum AJ 3170, Cetobacterium somerae ATCC BAA-474, Clostridium tyrobutyricum KCTC 5387, Oenococcus oeni PSU-1, Bacteroides caccae CL03T12C61, Clostridium cellulovorans 743B, Lactobacillus casei ATCC27139 and Veillonella parvula ATCC 17745.* This microbial consortium comprising the eight listed bacterial strains is referred herein as Consortium 3 ("Cons. 3"). Consortium 3 includes the microbial strains of both Consortium 1 and Consortium 2.

The microbial consortium may comprise at least one, at least two, at least three, at least all of *Cetobacterium somerae ATCC BAA-474, Clostridium tyrobutyricum KCTC 5387, Clostridium cellulovorans 743B and Veillonella parvula ATCC 17745.*

The microbial consortium may comprise *Cetobacterium somerae ATCC BAA-474, Clostridium tyrobutyricum KCTC 5387, Clostridium cellulovorans 743B and Veillonella parvula ATCC 17745.* This microbial consortium comprising the four listed bacterial strains is referred herein as Consortium 4 ("Cons. 4").

The microbial consortium may comprise at least one, at least two, at least three, at least all of *Cetobacterium somerae ATCC BAA-474, Clostridium tyrobutyricum KCTC 5387, Bacteroides caccae CL03T12C61and Veillonella parvula ATCC 17745.*

The microbial consortium may comprise *Cetobacterium somerae ATCC BAA-474, Clostridium tyrobutyricum KCTC 5387, Bacteroides caccae CL03T12C61and Veillonella parvula ATCC 17745.* This microbial consortium comprising the four listed bacterial strains is referred herein as Consortium 5 ("Cons. 5").

Also provided is a microbial consortium selected from Consortium 1, Consortium 2, Consortium 3, Consortium 4, and Consortium 5.

Also provided is a microbial consortium selected from Consortium 1, Consortium 2 and Consortium 3.

Also provided is a microbial consortium being at least one of Consortium 1, Consortium 2, Consortium 3, Consortium 4, and Consortium 5.

The microbial consortium may comprise the same or equivalent amounts of the microorganisms forming the consortium. The microbial consortium may comprise different amounts of the microorganisms forming the consortium. The total number (cell count/amount/ colony forming units-CFUs) of each one of microorganisms forming the microbial consortium may be between about 1X10³ to about 1X10¹², between 1X10⁵ to about 1X10¹⁰, 1X10⁸ to about 5X10¹⁰, between 2X10⁸ to about 4X10¹⁰, between 3X10⁸ to about 3X10¹⁰, between 5X10⁸ to about 1X10¹⁰.

The total number (cell count/amount/ colony forming units-CFUs) of microorganisms forming the microbial consortium may be between about 1X10³ to about 1X10¹², between 1X10⁵ to about 1X10¹⁰, 1X10⁸ to about 5X10¹⁰,, between 2X10⁸ to about 4X10¹⁰, between 3X10⁸ to about 3X10¹⁰, between 5X10⁸ to about 1X10¹⁰.

As detailed above, the two or more microorganisms can be identified in or isolated from the microbiome of a reference subject, for example by collecting a biological sample. The biological sample may be any sample from which the population of microorganisms can be isolated, for example, faeces. The sample may be a biopsy of human organs or tissue, specifically, a gut biopsy.

The microbial consortium may be formulated in a variety of forms, depending on the storage, administration etc. Non-limiting forms include solid, dry form, for example, in a lyophilized powder, gel form, a suspension, a cell lysate or extract. The microbial consortium may be suspended in a liquid medium (such as PBS or saline) and used in a suspension form.

The microbial consortium may be used in the preparation of a pharmaceutical formulation/compositions/suspension or in the manufacture of a formulation/compositions/suspension for use in treatment. As such, formulation/compositions/suspension may comprise apart from a therapeutically effective amount of a microbial consortium, at least one additional components as detailed herein.

Also provided is a composition (suspension or formulation) comprising microbial consortium. As described herein, the microbial consortium and/or the suspension/composition comprising the same may form a kit. In general, the composition and/or the suspension and/or kit described herein comprising the microbial consortium as well as the microbial consortium *per se* form part of this disclosure. It should be noted that the forms described herein for the microbial consortium *per se* apply for the composition and/or the suspension and/or kit comprising the microbial consortium.

The composition may optionally further comprise at least one of pharmaceutically acceptable carrier/s, excipient/s, additive/s diluent/s and adjuvant/s. As used herein "pharmaceutically acceptable carrier" includes any and all solvents, dispersion media, coatings and the like.

Aqueous suspensions may further contain substances which increase the viscosity of the suspension including, for example, sodium carboxymethylcellulose, sorbitol and/or dextran. The suspension may also contain stabilizers.

It should be understood that in addition to the ingredients particularly mentioned above, the formulations may also include other agents conventional in the art having regard to the type of formulation in question.

The microbial consortium of and/or any suspensions/compositions comprising the same can be administered and dosed by the methods as described below, in accordance with medical procedures known in the art. For example, the suspensions/compositions used in the methods and kits, described herein below, may be adapted for administration by various modes of interferon administration are known in the art. These include, but are not limited to, injection (e.g., using a subcutaneous, intramuscular, intravenous, or intradermal injection), intranasal administration and oral administration.

The microbial consortium and/or any suspensions/compositions comprising the same may be formulated for oral administration.

The microbial consortium and/or any suspensions/compositions comprising the same may be formulated for delivery to the intestine. In The microbial consortium and/or any suspensions/compositions comprising the same may be formulated into food or a beverage.

The microbial consortium and/or any suspensions/compositions comprising the same may be formulated to be contained within a carrier.

The microbial consortium and/or any suspensions/compositions comprising the same may be enterically coated.

As described herein, the microbial consortium, the compositions comprising the microbial consortium and the kits may be useful for a variety of purposes, such as in the treatment of subjects in need of a treatment by the microbial consortium. Specifically, by affecting the immune system of a host subject, treatment of, for example, a proliferative disorder may be achieved.

As described herein, the microbial consortium as well as compositions and kits of may be administered to a human subject for the treatment of proliferative disorder such as cancer. As shown in the examples below, the microbial consortiums were capable of inhibiting tumor growth.

Thus, further provided is a microbial consortium comprising two or more purified or isolated microorganisms, the compositions comprising the same or kits for use in a method of treating of a subject suffering from a proliferative disorder.

The microbial consortium may be for use in treating a subject being diagnosed with a disease that may be treatable by the microbial consortium as detailed herein, such as a proliferative disorder.

*A **proliferative disorder*** is a disorder displaying cell division and growth that is not part of normal cellular turnover, metabolism, growth, or propagation of the whole organism. Unwanted proliferation of cells is seen in tumors and other pathological proliferation of cells, does not serve normal function, and for the most part will continue unbridled at a growth rate exceeding that of cells of a normal tissue in the absence of outside intervention. A pathological state that ensues because of the unwanted proliferation of cells is referred herein as a "hyper-proliferative disease" or "hyper-proliferative disorder."

Non-limiting examples of proliferative disorder include cancer, atherosclerosis, rheumatoid arthritis, psoriasis, idiopathic pulmonary fibrosis, scleroderma and cirrhosis of the liver.

The microbial consortium may comprise two or more purified or isolated microorganisms, the compositions comprising the same and kits are for use in treating cancer.

As used herein, "cancer", "tumor" and "malignancy" all relate equivalently to a hyperplasia of a tissue or organ.

The microbial consortium composition or kits comprising the same may be used for the treatment of any one of non-solid tumor and solid tumors.

Non-limiting examples of cancers include blastoma, carcinoma, lymphoma, leukemia, sarcoma, mesothelioma, glioma, germinoma, choriocarcinoma, skin cancer (such as basal-cell skin cancer (BCC), squamous-cell skin cancer (SCC) melanoma), glioblastoma, lymphoid malignancies, squamous cell cancer (e.g. epithelial squamous cell cancer), lung cancer including small-cell lung cancer, non-small cell lung cancer, adenocarcinoma of the lung and squamous carcinoma of the lung, cancer of the peritoneum, hepatocellular cancer, gastric or stomach cancer including gastrointestinal cancer, pancreatic cancer, glioblastoma, cervical cancer, ovarian cancer, liver cancer, bladder cancer, hepatoma, breast cancer, colon cancer, rectal cancer, colorectal cancer, endometrial or uterine carcinoma, salivary gland carcinoma, kidney or renal cancer, prostate cancer, vulval cancer, thyroid cancer, hepatic carcinoma, anal carcinoma, penile carcinoma, as well as head and neck cancer.

The cancer may be a solid cancer type or a hematologic cancer type.

The cancer may be a solid cancer. The term solid cancer as used herein refers to a solid tumor is an abnormal mass of tissue that usually does not contain cysts or liquid areas and that is malignant. Examples of solid tumors are sarcomas, carcinomas, and lymphomas.

The cancer may be a hematologic cancer (also denoted as blood cancer). Hematologic cancer typically begins in blood-forming tissue, such as the bone marrow, or in the cells of the immune system. Examples of hematologic cancer are leukemia, lymphoma, and multiple myeloma.

The cancer may be at least one of breast cancer, lung cancer, ovarian cancer prostate cancer, liver cancer, pancreatic cancer, bladder cancer, prostate cancer, cancer of the uterine cervix, brain cancer, colon cancer, gastrointestinal cancer, skin cancer such as melanoma, lung cancer, kidney cancer, bladder cancer, head and neck cancer, and lymphomas.

The cancer may be a skin cancer. In some embodiments, the cancer is melanoma.

The cancer is at least one of breast cancer, lung cancer, pancreatic cancer, bladder cancer, melanoma, kidney cancer, head and neck cancer, and lymphoma.

In some embodiments, the cancer is selected from breast cancer, lung cancer, melanoma and kidney cancer.

In some embodiments, the cancer is selected from breast cancer and melanoma.

In some embodiments, the cancer is breast cancer.

In some embodiments, the cancer is lung cancer. The cancer may be a non-small cell lung cancer (NSCLC).

In some embodiments, the cancer is kidney cancer. In The cancer may be Renal Cell Carcinoma (RCC).

In some embodiments, the cancer is bladder cancer.

The cancer may be prostate cancer.

In some embodiments, the cancer is pancreatic cancer.

In some embodiments, the cancer is head and neck cancer.

In some embodiments, the cancer is lymphoma.

The microbial consortium comprising two or more purified or isolated microorganisms may be administrated in combination with an additional treatment.

As shown in examples below (e.g. example 2), the microbial consortium were superior over anti PD-1 inhibitor alone in their ability to inhibit tumor growth as well as in their ability to modulate the activity, expression and secretion of pro-inflammatory cytokines and cluster of differentiation molecules. Specifically, treatment with the microbial consortium induced an increased secretion of plasma cytokines, INFg, IL6, IL12 and an increased expression of CD8 and CD68 as compared to treatment with anti PD-1 alone.

In other words, the microbial consortium may be used as for treatment alone or as an adjuvant therapy, specifically for treating cancer.

The microbial consortium may be administered in simultaneously or sequentially with an anticancer treatment.

The microbial consortium may be administered concurrently with an anticancer treatment.

Both the microbial consortium and the anticancer treatment may be administered any administration method and route commonly known and/or used in the field, which may be the same or different for the two.

Non-limiting examples of an anti-cancer agent and/or anti-cancer treatment include chemotherapy, radiation therapy, surgical therapy, a receptor kinase inhibitor (such as tyrosine), an immune checkpoint inhibitor or any other targeted cancer therapy, an immunotherapy agent, a hormone agent, a biological agent, cytokine agent, a differentiation factor, an anti-angiogenic factor, CAR T-cell therapy or any other cell-based therapy, or an immune-modulatory.

The anticancer treatment may be an anticancer agent.

The anticancer treatment may be a receptor kinase inhibitor (such as tyrosine), an immune checkpoint inhibitor or any other targeted cancer therapy, an immunotherapy agent, a hormone agent, a biological agent, cytokine agent, a differentiation factor, an anti-angiogenic factor, CAR T-cell therapy or any other cell-based therapy, or an immune-stimulatory

The anticancer treatment may be an immune checkpoint inhibitor.

An immune checkpoint inhibitor typically block certain proteins made by some types of immune system cells, such as T cells, and some cancer cells. Examples of checkpoint proteins found on T cells or cancer cells include, for example, PD-1/PD-L1 and CTLA-4/B7-1/B7-2. A checkpoint inhibitor may be an immunotherapy agent such as for example a monoclonal antibody.

The anticancer treatment may be a checkpoint inhibitor selected from an anti- PD-1 inhibitor, an anti-CTLA-4 inhibitor, an anti-PD-L1 inhibitor or combinations thereof. The inhibitor may be a small molecule, an antibody, an aptamer, an oligomer, a polymer.

The checkpoint inhibitor may be selected from an anti- PD-1 antibody, an anti-CTLA-4 antibody, an anti-PD-L1 antibody or combinations thereof.

The checkpoint inhibitor may be at least one of Ipilimumab, Nivolumab, Pembrolizumab, Atezolizumab, Avelumab, Durvalumab and Cemiplimab or any combinations thereof.

The checkpoint inhibitor is an anti- PD-1 inhibitor.

The checkpoint inhibitor may be Pembrolizumab.

As shown in the examples below the microbial consortium reduces the rate of tumor growth. Hence, it was suggested that the microbial consortium can be used in method of inhibiting cancer growth and hence in cancer treatment.

Also provided is a method for treating a disorder in a subject in need thereof. The method for treating, preventing, ameliorating, reducing or delaying the onset of a proliferative disorder in a subject in need thereof may comprise administering to such subject a therapeutically effective amount of a microbial consortium or any composition or kit comprising the same.

In accordance with the methods, administering the effective amount of a microbial consortium ameliorates one or more signs or symptoms of the proliferative disorder, such as cancer. In other words, the methods are for treating disorders may be treatable with the microbial consortium.

Also provided is a method of treating, preventing, ameliorating, reducing or delaying the onset of a proliferative disorder, such as cancer, in a human subject in need thereof comprising the step of administering to the subject an effective amount of a microbial consortium comprising two or more isolated microorganism or purified microorganism, at least one of the two or more microorganisms may be present at low-abundance in a microbiome of a reference human subject. The microbial consortium may comprise at least one other of the two or more microorganisms having at least one of (i) capable of modulating at least one SCFA, (ii) capable of modulating lactate and (iii) comprises at least one cell envelope component being recognized by the human subject's immune system or an epithelial cell, such as intestinal epithelial cell.

Also provided is a method of treating, preventing, ameliorating, reducing or delaying the onset of a proliferative disorder, such as cancer, in a human subject in need thereof comprising the step of administering to the subject an effective amount of a microbial consortium comprising two or more isolated microorganism or purified microorganism, at least one of the two or more microorganisms may be present at low-abundance in a microbiome of a reference human subject and having at least one (i) capable of modulating at least one SCFA, (ii) capable of modulating lactate and (iii) comprises at least one cell envelope component being recognized by the human subject's immune system or an epithelial cell, such as intestinal epithelial cell.

The methods may comprise administration of a therapeutically effective amount of a microbial consortium comprises 16S rRNA sequences having at least at least 85%, at least 90%, at least 95%, at least 96%, at least 97%, at least 98%, at least 99%, at least 99.5% identity with nucleic acid sequences selected from the group consisting of SEQ ID NO: 1, SEQ ID NO:2, SEQ ID NO:3, SEQ ID NO:4 and SEQ ID NO:5.

The methods may comprise administration of a therapeutically effective amount of a microbial consortium comprises one, two, three, four or five microorganisms selected from *Oenococcus oeni* PSU-1, *Clostridium tyrobutyricum* KCTC 5387, *Cetobacterium somerae* ATCC BAA-474, *Corynebacterium glyciniphilum* AJ 3170, and *Clostridium cellulovorans 743B.*

The methods may comprise administration of a therapeutically effective amount of a microbial consortium comprises at least one microorganism, at least two microorganisms, at least three microorganisms from the *Bacteroides caccae* species, *Lactobacillus casei* species or *Veillonella parvula* species.

The methods may comprise administration of a therapeutically effective amount of a microbial consortium comprises at least one microorganism comprises 16S rRNA sequences having between 85% to 99%, at times between 90% to 99%, at times between 95% to 99%, at times between 96% to 99%, at times between 97% to 99%, at times between 98% to 99% identity with nucleic acid sequences selected from the group consisting of SEQ ID NO:7, SEQ ID NO:8 and SEQ ID NO:9.

The methods may comprise administration of a therapeutically effective amount of a microbial consortium comprises at least one, at least two of *Bacteroides caccae* CL03T12C61, *Lactobacillus casei A TCC2 7139* or *Veillonella parvula ATCC 17745.*

The methods of may comprise administration to the subject a therapeutically effective amount of a microbial consortium comprising two or more purified or isolated microorganisms having a 16S rDNA sequence that is at least 95%, at least 96%, at least 97%, at least 98%, at least 99%, at least 99.5% identical to one, two, three, four, five, six seven, eight 16S rDNA sequence listed in Table 1.

The methods may comprise administration to the subject a therapeutically effective amount of a microbial consortium comprising two or more purified or isolated microorganisms having a 16S rDNA sequence that is at least 95%, at least 96%, at least 97%, at least 98%, at least 99%, at least 99.5% identical to one, two, three, four, five, six seven, eight 16S rDNA sequence denoted by of SEQ ID NO:1, SEQ ID NO:2, SEQ ID NO:3, SEQ ID NO:4, SEQ ID NO:5, SEQ ID NO:6, SEQ ID NO:7 and SEQ ID NO:8.

The methods may comprise administration to the subject a therapeutically effective amount of a microbial consortium being at least one of Consortium 1, Consortium 2, Consortium 3, Consortium 4, and Consortium 5.

Also provided is a method for treating, preventing, ameliorating, reducing or delaying the onset of a proliferative disorder, such as cancer, in a human subject in need thereof comprising administration a therapeutically effective amount of the microbial consortium with a therapeutically effective amount of an anticancer treatment.

The method may comprise administrating to the subject a therapeutically effective amount of the microbial consortium and a therapeutically effective amount of the anticancer treatment simultaneously or sequentially.

The method may comprise administrating to the subject a therapeutically effective amount of the microbial consortium and a therapeutically effective amount of the anticancer treatment concurrently.

The methods for treating, preventing, ameliorating, reducing or delaying the onset of cancer, may comprise administrating to the subject in need thereof a therapeutically effective amount of the microbial consortium and a therapeutically effective amount of the anticancer treatment, the method comprising administrating a therapeutically effective amount of the microbial consortium with a therapeutically effective amount of a checkpoint inhibitor as detailed herein.

The cancer may be at least one of breast cancer, lung cancer, ovarian cancer prostate cancer, liver cancer, pancreatic cancer, bladder cancer, prostate cancer, cancer of the uterine cervix, skin cancer (such as melanoma), brain cancer, colon cancer, gastrointestinal cancer, melanoma, lung cancer, kidney cancer, bladder cancer, head and neck cancer, and lymphomas.

The cancer is at least one of breast cancer, lung cancer, pancreatic cancer, bladder cancer, melanoma, kidney cancer, head and neck cancer, and lymphoma.

In some embodiments, the cancer is selected from breast cancer, lung cancer, melanoma and kidney cancer.

In some embodiments, the cancer is selected from breast cancer and melanoma.

The cancer may be a skin cancer. In some embodiments, the cancer is melanoma.

In some embodiments, the cancer is breast cancer.

In some embodiments, the cancer is lung cancer. The cancer may be a non-small cell lung cancer (NSCLC).

In some embodiments, the cancer is kidney cancer. The cancer may be Renal Cell Carcinoma (RCC).

In some embodiments, the cancer is bladder cancer.

The cancer may be prostate cancer.

In some embodiments, the cancer is pancreatic cancer.

In some embodiments, the cancer is head and neck cancer.

In some embodiments, the cancer is lymphoma.

The microbial consortium may be administrated to a human subject by any method known in the art and described herein. The methods ,may comprise administration the microbial consortium or a composition comprising the microbial consortium by oral administration. The methods may comprise administration the microbial consortium or a composition comprising the microbial consortium that is formulated for delivery to the intestine. The microbial consortium or a composition comprising the microbial consortium may be formulated for example into a capsule, tablet, food or beverage or any of the like.

The methods may comprise administrating the microbial consortium, compositions or kits comprising the same and optionally an additional treatment, as a single one-time dose, as a single daily dose or multiple daily doses, preferably, every 1 to 7 days. It is specifically contemplated that such application may be carried out once or several times in the lifetime of a patient, once, twice, thrice, four times, five times or six times daily, or may be performed once daily, once every 2 days, once every 3 days, once every 4 days, once every 5 days, once every 6 days, once every week, two weeks, three weeks, four weeks or even more than a month.

Further provided is the use of a microbial consortium in the preparation of a composition for treating a proliferative disorder in a subject in need thereof. Thus, there is provided a use of a microbial consortium comprising two or more microorganisms, at least one of the two or more microorganisms may be present at low-abundance in a microbiome of a reference human subject and at least one other of the two or more microorganisms having at least one of (i) capable of modulating at least one short chain fatty acid (SCFA), (ii) capable of modulating lactate and (iii) comprises at least one cell envelope component in the preparation of a composition for treating a proliferative disorder in a subject in need thereof.

Further provided is the use of a microbial consortium in the preparation of a composition for treating a proliferative disorder in a subject in need thereof. Thus, there is provided a use of a microbial consortium comprising two or more microorganisms, at least one of the two or more microorganisms may be present at low-abundance in a microbiome of a reference human subject and having at least one (i) capable of modulating at least one SCFA, (ii) capable of modulating lactate and (iii) comprises at least one cell envelope component in the preparation of a composition for treating a proliferative disorder in a subject in need thereof.

Also provided is a kit comprising microbial consortium in the preparation of a composition for treating a proliferative disorder in a subject in need thereof. Thus, there is provided a use of a microbial consortium comprising two or more microorganisms, at least one of the two or more microorganisms may be present at low-abundance in a microbiome of a reference human subject and at least one other of the two or more microorganisms having at least one of (i) capable of modulating at least one short chain fatty acid (SCFA), (ii) capable of modulating lactate and (iii) comprises at least one cell envelope component.

Also provided is a kit comprising microbial consortium in the preparation of a composition for treating a proliferative disorder in a subject in need thereof. Thus, there is provided a use of a microbial consortium comprising two or more microorganisms, at least one of the two or more microorganisms may be present at low-abundance in a microbiome of a reference human subject and having at least one (i) capable of modulating at least one SCFA, (ii) capable of modulating lactate and (iii) comprises at least one cell envelope component.

The kits described herein may include a composition/suspension as described, as an already prepared dosage form ready for administration or, alternatively, can include the microbial consortium or a composition comprising the microbial consortium as described as a solid pharmaceutical composition (e.g. in a lyophilized form) that can be reconstituted with a solvent to provide a liquid dosage form. The kit may additionally comprise instructions for using the kit in treating a proliferative disorder.

The methods of treatment provided herein involve administration of the microbial consortium in a therapeutically effective amount. The term "effective amount" as used herein is that determined by such considerations as are known to the man of skill in the art.

Provided are methods for treating or preventing a proliferative disorder. The term "treatment or prevention" refers to the complete range of therapeutically positive effects of administrating to a subject including inhibition, reduction of, alleviation of, and relief from, proliferative disorder symptoms or undesired side effects of such proliferative disorder related disorders.

As used herein, "disease", "disorder", "condition" and the like, as they relate to a subject's health, are used interchangeably and have meanings ascribed to each and all of such terms.

The present disclosure relates to methods for the treatment of subjects, or patients, in need thereof. By "patient" or "subject in need" it is meant any organism who may be affected by the above-mentioned conditions, and to whom the treatment methods herein described are desired. It should be further noted that particularly in case of human subject, administering of the compositions to the patient includes both self-administration and administration to the patient by another person.

Provided are methods for treating proliferative disorders, and further relates to disorders associated or related to cancer. It is understood that the interchangeably used terms "associated" and "related", when referring to pathologies herein, mean diseases, disorders, conditions, or any pathologies which at least one of: share causalities, co-exist at a higher than coincidental frequency, or where at least one disease, disorder condition or pathology causes the second disease, disorder, condition or pathology.

Wherein indicated "increasing" or "enhancing" the activity by means of modulation, as used herein in connection with the microbiome consortium, may mean that such increase or enhancement may be an increase or elevation of between about 5% to 100%, specifically, 10% to 100% of the activity as compared to a suitable control for example being no treatment, for example with no stimulant (no microorganisms, denoted herein as -LPS) or without an additional anticancer drug, such as without an anti-PD1, or in the absence of the microbiome consortium.

The term "about" as used herein indicates values that may deviate up to 1%, more specifically 5%, more specifically 10%, more specifically 15%, and in some cases up to 20% higher or lower than the value referred to, the deviation range including integer values, and, if applicable, non-integer values as well, constituting a continuous range. As used herein the term "about" refers to ± 10 %.

**Table 1- List of Sequences**

| **SEQ ID NO:** | | **Details** |
|---|---|---|
| | 1 | Gene encoding 16S rRNA of *Oenococcus oeni* PSU-1 |
| | 2 | Gene encoding 16S rRNA of *Clostridium tyrobutyricum* KCTC 5387 |
| | 3 | Gene encoding 16S rRNA of *Cetobacterium somerae* ATCC BAA-474 |
| | 4 | Gene encoding 16S rRNA of *Corynebacterium glyciniphilum* AJ 3170 |
| | 5 | Gene encoding 16S rRNA of *Clostridium cellulovorans 743B* |
| | 6 | Gene encoding 16S rRNA of Methanosphaera stadtmanae DSM 3091 |
| | 7 | Gene encoding 16S rRNA of *Veillonella parvula ATCC 17745* |
| | 8 | Gene encoding 16S rRNA of *Bacteroides caccae* CL03T12C61 |
| | 9 | Gene encoding 16S rRNA of *Lactobacillus casei ATCC27139* |
| | 10 | Gene encoding 16S rRNA of *Ruminococcus albus 7* |
| | 11 | Gene encoding 16S rRNA of *Bifidobacterium breve* ATCC15700 |
| | 12 | Gene encoding 16S rRNA of *Alistipes putredini* DSM 17216 |
| | 13 | Gene encoding 16S rRNA of *Akkermansia muciniphila* ATCC BAA-835 |
| | 14 | Gene encoding 16S rRNA of *Akkermansia muciniphila* YL44 |

### Non-limiting examples:

### Example 1: in vitro studies

### Example 1A: Toll-like receptors (TLR) activation

The objective of this study was to evaluate TLR activation following incubation with various bacterial strains, using InvivoGen's HEK-Blue^{™} TLR cells (derived from HEK293 cells) and secreted alkaline phosphatase (SEAP) reporter system.

### Materials and methods

HEK-Blue^{™} TLR2 and HEK-Blue^{™} TLR4 cells were incubated with escalating concentrations of various bacterial strains (bacteria:cell ratios 1:2.78, 1:8.33, 1:5) for 20 hours at 37°C, 5% CO₂. TLR activation was measured as SEAP secretion and quantified using colorimetric reader in 630nm OD, as follows, TLR2 and TLR4 ligands activate NF-κB and AP-1 which induce the production of SEAP. HEK-Blue^{™} Detection cell culture medium allows the detection of SEAP as a readout of TLR activation.

### Results

As can be seen from **Figs. 1A to 1E** and **Figs. 2A to 2C****,** all the tested bacterial strains at all the tested ratios activated TLR4 and TLR2. Specifically, a large increase vs. LPS (being the positive control) was observed in TLR4 activation with *Cetobacterium somerae* ATCC BAA-474 **(****Fig. 1C****)** and in TLR2 activation with *Oenococcus oeni* PSU-1 and *Corynebacterium glyciniphilum* AJ 3170 **(****Figs. 2B** and **2C****).** These results suggested that the tested bacterial strains and specifically the low abundance bacterial strains were recognized by the specific TLR and in turn, activated the immune cell response.

### Example 1B: Cytokines secretion in macrophage cells lines

The objective of this study was to evaluate the efficacy of various bacterial strains and combinations of bacterial strains in stimulating cytokines' secretion from RAW murine macrophages cell lines, compared to Lipopolysaccharide (LPS) stimulation.

### Materials and methods

RAW cells were incubated with various bacterial strains (10⁸ CFU/ml) for 16 hours at 37°C, 5% CO_{2.} The medium (supernatant) was collected and analyzed for cytokines secretion by R&D DouSet ELISA development systems according to manufacturer's protocol. Each bacteria was tested in triplicates.

### Results

The effect of individual bacterial strains on cytokine secretion showed that all the tested microorganisms were capable of inducing cytokines secretion from macrophages, as compared to no simulant (-LPS) **(****Figs. 3A -3C****).** **Figs. 3A** and **3B** show that *Cetobacterium somerae* ATCC BAA-474 induced the largest secretion of TNFα and IL6 and **Fig. 3C** shows that *Veillonella parvula ATCC 17745,* followed by *Cetobacterium somerae* ATCC BAA-474 induced the largest secretion of IL1b.

All the tested bacterial strains have the ability to induce proinflammatory cytokines (TNFα, IL-6, IL-1β) in macrophage cell line in a higher level than LPS stimulation
These data taken collectively suggest an important role of the identified bacterial strains and specifically the low abundance bacterial strains in inducting cytokine secretion from macrophages.

It was also found that various combinations of two bacterial strains induced secretion of cytokines, suggesting that the combination of two bacterial strains may increase the signal of pro-inflammatory response (data not shown).

The effect of various combinations of bacterial on secretion of cytokines was further tested. **Figs. 4A to 4C** show the effect of bacterial combinations in inducing secretion of cytokines. Specifically, the following five combinations (consortiums) were tested:
Consortium 1 ("Cons. 1") includes *Corynebacterium glyciniphilum* AJ 3170, *Cetobacterium somerae* ATCC BAA-474, *Clostridium tyrobutyricum* KCTC 5387 and *Oenococcus oeni* PSU-1;
Consortium 2 ("Cons. 2") comprises *Bacteroides caccae* CL03T12C61, *Clostridium cellulovorans 743B, Lactobacillus casei ATCC27139, Veillonella parvula ATCC 17745;*
Consortium 3 ("Cons. 3") comprises *Corynebacterium glyciniphilum* AJ 3170, *Cetobacterium somerae* ATCC BAA-474, *Clostridium tyrobutyricum* KCTC 5387, *Oenococcus oeni* PSU-1, *Bacteroides caccae* CL03T12C61, *Clostridium cellulovorans 743B, Lactobacillus casei ATCC27139* and *Veillonella parvula ATCC 17745;*
Consortium 4 ("Cons. 4") comprises *Cetobacterium somerae* ATCC BAA-474, *Clostridium tyrobutyricum* KCTC 5387, *Clostridium cellulovorans 743B* and *Veillonella parvula ATCC 17745;*
Consortium 5 ("Cons. 5") comprises *Cetobacterium somerae* ATCC BAA-474, *Clostridium tyrobutyricum* KCTC 5387, *Bacteroides caccae* CL03T12C61and *Veillonella parvula ATCC 17745.*

The results in **Figs. 4A to 4C** show that all the tested bacterial consortiums induced secretion of cytokines suggesting that combination of the identified bacterial strains are effective in modulating proinflammatory response. This may suggests an additive effect of the bacterial strains in each consortium.

### Example 1C: Cytokines secretion from human peripheral blood mononuclear cell (PBMCs)

The objective of this study was to evaluate the efficacy of various bacterial strains and combinations of bacterial strains in stimulating cytokines' secretion from PBMC, compared to Lipopolysaccharide (LPS) stimulation.

### Materials and methods

Human Venous blood (10-15 mL) was collected into heparinized vials and mixed well by gently inverting the tube several times. The blood was diluted x2 with PBS. Histopaque was added to a 50 mL centrifuge tube in half of the final blood: PBS volume. The blood was gently layered on the top of Histopaque using a 1 mL auto pipette. The layering was done very slowly so that blood and Histopaque stayed as two different layers. The tubes were centrifuged (without any delay) at 400 x g for 30 min at +20°C in a swing-out bucket without break. The whitish buffy coat (PBMCs) formed in the interphase between Histopaque and medium was aspirated without any delay. The cells were washed (centrifuged in 800 x g for 10 min at +20°C) twice with 10 mL of PBS.

PBMCs were incubated with each bacterial strain in final concentration of 10^8 CFU/ml or Vehicle (10% PBS) or Positive control (10 ng/mL LPS) for 48 hours. Following incubation period, supernatant was collected and analyzed for cytokines secretion by R&D DouSet ELISA development systems according to manufacturer's protocol. Each bacteria was tested in triplicates.

### Results

**Figs. 5A-5B** are bar graphs showing the efficacy of bacterial strains in stimulating cytokines' secretion from PBMCs, IL6 **(****Fig. 5A****),** TNFα **(****Fig. 5B****).** As can be seen, most of the bacterial strains including the low abundance bacterial strains stimulated secretion from the cells in an increased level as compared with LPS secretion.

Taken together, the in vitro results show the ability of the bacterial strains as well as the combination of two or more bacterial strains to increase the signal of pro-inflammatory response both from macrophages and from PBMC.

Interestingly, the low abundance bacterial strains were capable of activating the proinflammatory response as good or even to a higher extend compared with the high abundance bacterial strains suggesting that although these bacterial strains are present in the microbiome at low abundance, they have the potential to activate an inflammatory response in the host.

This type of inflammatory response is suggested to suppress and limit tumor growth.

### Example 2: in vivo studies of mice bearing breast cancer tumors

The objective of the study was to determine the efficacy of bacterial consortia treatment in combination with anti PD-1 treatment (Anti-Mouse PD-1 (CD279), which is the equivalent of pembrolizumab for use in murine) on the development of E0771 derived breast cancer tumors in C57BL/6 female mice.

### Materials and methods

On study *"Day 1*", mice were injected into their right flank with 1.5x10⁵ E0771 cells in 50 µL PBS:Matrigel (1:1). Tumor volume was measured from Day 5 and twice a week thereafter until study termination. In addition, on Day 1 the mice started receiving antibiotics cocktail (Ampicillin (1 g/L), Neomycin (1 g/L), Metronidazole (1 g/L) and Vancomycin (0.5 g/L)) in their drinking water. Antibiotics were administered until and including Day 13. On Day 14 of the study the water and water bottles were changed to water without antibiotics.

On Day 15 of the study, mice were allocated into five groups of 10 or 15 mice, with similar mean tumor volume. The first oral treatment was administered 24 hours after cessation of antibiotics; the bacterial consortia were administered by oral gavage and anti-PD1 by intraperitoneally administration. Thus, on day "15" of the study, mice were administered with one of these treatments; (1) anti-PD1, (2) Cons. 1+ anti-PD1, (3) Cons. 2+ anti-PD1 or (4) Cons. 3+ anti-PD1, (5) PBS = administrated via oral gavage at similar volume used to administer bacterial consortia as control group. During the study, mice received another seven oral gavages of bacteria (2 gavages per week) and anti-PD1 or only anti-PD1.

The microbial combination used in this study were as follows:
Consortium 1 ("Cons. 1") includes *Corynebacterium glyciniphilum* AJ 3170, *Cetobacterium somerae* ATCC BAA-474, *Clostridium tyrobutyricum* KCTC 5387 and *Oenococcus oeni* PSU-1;
Consortium 2 ("Cons. 2") comprises *Bacteroides caccae* CL03T12C61, *Clostridium cellulovorans 743B, Lactobacillus casei ATCC27139, Veillonella parvula ATCC 17745;*
Consortium 3 ("Cons. 3") comprises *Corynebacterium glyciniphilum* AJ 3170, *Cetobacterium somerae* ATCC BAA-474, *Clostridium tyrobutyricum* KCTC 5387, *Oenococcus oeni* PSU-1, *Bacteroides caccae* CL03T12C61, *Clostridium cellulovorans 743B, Lactobacillus casei ATCC27139* and *Veillonella parvula ATCC 17745.*

Tumors were measured for length (L) and width (W) using a digital caliper starting from tumor appearance (around 4 days after cell inoculation) and twice a week thereafter. Tumor volume (in mm³) was calculated according to the equation (L×W²×0.5).

Th1 cytokine panel was evaluated on plasma samples (IFNγ, IL-6 and IL-12) taken at the day of termination of each animal.

At the end of the study, tumors were excised and placed in formalin and further processed for histopathological evaluation. Paraffin-embedded 4µm section of the tumors were stained with H&E and examined for inflammation parameters and infiltration of lymphocytes that were graded by an acceptable grading system. In addition, immunohistochemistry (IHC) analyses was performed to detect CD8 and CD68 expression using specific antibodies.

The following parameters were evaluated:
The inflammatory infiltrate within or at the periphery of the nodule were evaluated and characterized as to its intensity and the inflammation was scored 0-3 (0 = scant or absent; 1 = inflammatory cells obviously present but markedly less than other cells; 2 = inflammatory cells roughly equal to melanocytes; 3 = predominantly inflammatory cells).

Lymphocytes infiltration within the tumor was graded on a scale of 0-3 (0 = absence of lymphocytes in the tumor; 1 = < 5 lymphocyte per high power field (HPF); 2 = >5 and < 20 lymphocytes per HPF 3 = > 20 lymphocytes per HPF).

The IHC (immunohistochemistry) scoring grade (CD8, CD68) was held performed on a scale of 0 to 4:
0= No cells were detected
1= Very few cells were detected (1-5 per X10 magnification)
2= Few cells were detected (6-15 per X10 magnification)
3= Moderate number of cells were detected (16-50 per X10 magnification)
4= Many cells were detected (more than 50 per X10 magnification)

### Results

**Fig. 6A** shows a reduction in tumor volume for all the three bacterial strains combinations administrated with anti-PD1 as compared to untreated mice.

The results shown in **Fig. 6A** also show that all the three bacterial strains combinations administrated with anti-PD1 were more effective in reducing tumor volume in comparison with anti-PD1 alone. As can be seen, the effect of consortium 1 with anti-PD1 and consortium 2 with anti-PD1 was observed at later time points. This may be attributed for example to the time period required for colonialization of the bacterial strains combinations and activation of the host immune system by the bacterial strains combinations.

The results of **Fig. 6A** also show that the effect on tumor volume of consortium 3 that comprise 8 bacterial strains with anti-PD1 was more significant and was observed at an earlier time point as compared to consortium 1 with anti-PD1 and consortium 2 with anti-PD1.

Taken together, these results suggest that the combination of the bacterial strains induce an additive pro-inflammatory response that in turn suppress tumor growth.

These results are further supported in **Fig. 6B****,** accordingly, the time to reach the study end point (being a tumor volume of 1000 mm²) was longer for breast tumors in mice treated with some combination of the bacterial strains an anti PD-1 as compared to treatment only with anti-PD1.

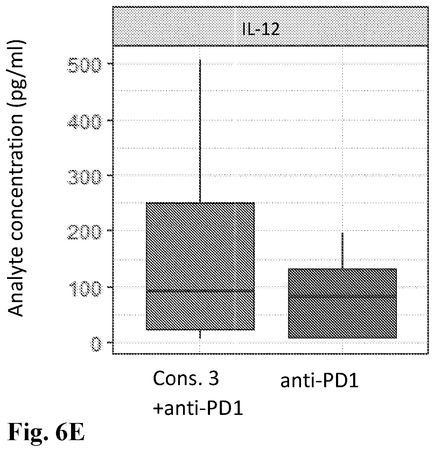

Further, analysis of various cytokines in the plasma of the mice showed that the combination of the bacterial strains an anti PD-1 induced an increased secretion of plasma cytokines, INFγ, (Fig. 6C), IL6 (Fig. 6D), IL12 (Fig. 6E) as compared to mice treated with anti PD-1 alone.

Histological evaluation of the tumors was in correlation with the increased proinflammatory response induced by the combination of the bacterial strains an anti PD-1. Specifically, the expression of CD8 and CD68 was increased in the tumors of mice treated with the combination of the bacterial strains with an anti PD-1 as compared to mice treated with anti PD-1 alone. This pattern of response was also evident in the inflammation score.

### Example 3: in vivo studies of mice bearing breast cancer tumors

The objective of the study is to determine the efficacy of bacterial consortia treatment in combination with anti PD-1 treatment on the development of E0771 derived breast cancer tumors in C57BL/6 female mice.

### Materials and methods

On study *"Day 1*", mice are injected into their right flank with 1.5x10⁵ E0771 cells in 50 µL PBS:Matrigel (1:1). Tumor volume is measured from Day 5 and twice a week thereafter until study termination. In addition, on Day 1 the mice receive antibiotics cocktail (Ampicillin (1 g/L), Neomycin (1 g/L), Metronidazole (1 g/L) and Vancomycin (0.5 g/L)) in their drinking water. Antibiotics are administered until and including Day 13. On Day 14 of the study the water and water bottles are changed to water without antibiotics.

On Day 15 of the study, mice are allocated into groups of 10 or 15 mice, with similar mean tumor volume. The first oral treatment is administered 24 hours after cessation of antibiotics; the bacterial consortia are administered by oral gavage and anti-PD1 by i.p administration. Thus, on day "15" of the study, mice are administered with one of these treatments; (1) anti-PD1, (2) Cons. 4+ anti-PD1, (3) Cons. 5+ anti-PD1 or (4) PBS = administrated via oral gavage at similar volume is used to administer bacterial consortia as control group. During the study, mice receive another seven oral gavages of bacteria (2 gavages per week) and anti-PD1 or only anti-PD1.

Consortium 4 ("Cons. 4") comprises *Cetobacterium somerae* ATCC BAA-474, *Clostridium tyrobutyricum* KCTC 5387, *Clostridium cellulovorans 743B* and *Veillonella parvula ATCC 17745;*

Consortium 5 ("Cons. 5") comprises *Cetobacterium somerae* ATCC BAA-474, *Clostridium tyrobutyricum* KCTC 5387, *Bacteroides caccae* CL03T12C61and *Veillonella parvula ATCC 17745.*

Tumors are measured for length (L) and width (W) using a digital caliper starting from tumor appearance (around 4 days after cell inoculation) and twice a week thereafter. Tumor volume (in mm³) is calculated according to the equation (L×W²×0.5).

Th1 cytokine panel is evaluated on plasma samples (IFNγ, IL-6 and IL-12) taken at the day of termination of each animal.

At the end of the study, tumors are excised and placed in formalin and are further process for histopathological evaluation. Paraffin-embedded 4µm section of the tumors are stained with H&E and are examine for inflammation parameters and infiltration of lymphocytes that are graded by an acceptable grading system. In addition, immunohistochemistry (IHC) analyses is performed to detect CD8 and CD68 expression using specific antibodies.

The following parameters are evaluated:
The inflammatory infiltrate within or at the periphery of the nodule are evaluated and characterized as to its intensity and the inflammation is scored 0-3 (0 = scant or absent; 1 = inflammatory cells obviously present but markedly less than other cells; 2 = inflammatory cells roughly equal to melanocytes; 3 = predominantly inflammatory cells).

Lymphocytes infiltration within the tumor is graded on a scale of 0-3 (0 = absence of lymphocytes in the tumor; 1 = < 5 lymphocyte per high power field (HPF); 2 = >5 and < 20 lymphocytes per HPF 3 = > 20 lymphocytes per HPF).

The IHC (immunohistochemistry) scoring grade (CD8, CD68) is held performed on a scale of 0 to 4:
0= No cells are detected
1= Very few cells are detected (1-5 per X10 magnification)
2= Few cells are detected (6-15 per X10 magnification)
3= Moderate number of cells are detected (16-50 per X10 magnification)
4= Many cells are detected (more than 50 per X10 magnification)

### Example 4: in vivo studies of mice bearing melanoma

The objective of the study is to determine the efficacy of bacterial consortia treatment in combination with anti PD-1 treatment on the development of melanoma tumors in C57BL/6 mice.

### Materials and methods

On study *"Day* 1", mice are injected into their right flank with 1.5x10⁵ BRAF KO cells in 50 µL PBS:Matrigel (1: 1). Tumor volume is measured from Day 5 and twice a week thereafter until study termination. In addition, on Day 1 the mice receive antibiotics cocktail (Ampicillin (1 g/L), Neomycin (1 g/L), Metronidazole (1 g/L) and Vancomycin (0.5 g/L)) in their drinking water. Antibiotics are administered until and including Day 13. On Day 14 of the study the water and water bottles are changed to water without antibiotics.

On Day 15 of the study, mice are allocated into groups of 10 or 15 mice, with similar mean tumor volume. The first oral treatment is administered 24 hours after cessation of antibiotics; the bacterial consortia are administered by oral gavage and anti-PD1 by i.p administration. Thus, on day "15" of the study, mice are administered with one of these treatments; (1) anti-PD1, (2) Cons. 1+ anti-PD1, (3) Cons. 2+ anti-PD1 or (4) Cons. 3+ anti-PD1, (5) Cons. 4+ anti-PD1, (6) Cons. 5+ anti-PD1 (7) PBS = administrated via oral gavage at similar volume that is used to administer bacterial consortia as control group.

During the study, mice receive another seven oral gavages of bacteria (2 gavages per week) and anti-PD1 or only anti-PD1.

The microbial combination to be used in this study are as follows:
Consortium 1 ("Cons. 1") includes *Corynebacterium glyciniphilum* AJ 3170, *Cetobacterium somerae* ATCC BAA-474, *Clostridium tyrobutyricum* KCTC 5387 and *Oenococcus oeni* PSU-1;
Consortium 2 ("Cons. 2") comprises *Bacteroides caccae* CL03T12C61, *Clostridium cellulovorans 743B, Lactobacillus casei ATCC27139, Veillonella parvula ATCC 17745;*
Consortium 3 ("Cons. 3") comprises *Corynebacterium glyciniphilum* AJ 3170, *Cetobacterium somerae* ATCC BAA-474, *Clostridium tyrobutyricum* KCTC 5387, *Oenococcus oeni* PSU-1, *Bacteroides caccae* CL03T12C61, *Clostridium cellulovorans 743B, Lactobacillus casei ATCC27139* and *Veillonella parvula ATCC 17745.*
Consortium 4 ("Cons. 4") comprises *Cetobacterium somerae* ATCC BAA-474, *Clostridium tyrobutyricum* KCTC 5387, *Clostridium cellulovorans 743B* and *Veillonella parvula ATCC 17745;*
Consortium 5 ("Cons. 5") comprises *Cetobacterium somerae* ATCC BAA-474, *Clostridium tyrobutyricum* KCTC 5387, *Bacteroides caccae* CL03T12C61and *Veillonella parvula ATCC 17745.*

Tumors are measured for length (L) and width (W) using a digital caliper starting from tumor appearance (around 4 days after cell inoculation) and twice a week thereafter. Tumor volume (in mm³) is calculated according to the equation (L×W²×0.5).

Th1 cytokine panel is evaluated on plasma samples (IFNγ, IL-6 and IL-12) taken at the day of termination of each animal.

At the end of the study, tumors are excised and are placed in formalin and are further processed for histopathological evaluation. Paraffin-embedded 4µm section of the tumors are stained with H&E and are examined for inflammation parameters and infiltration of lymphocytes that are graded by an acceptable grading system. In addition, immunohistochemistry (IHC) analyses is performed to detect CD8 and CD68 expression using specific antibodies.

The following parameters are evaluated:
The inflammatory infiltrate within or at the periphery of the nodule are evaluated and characterized as to its intensity and the inflammation is scored 0-3 (0 = scant or absent; 1 = inflammatory cells obviously present but markedly less than other cells; 2 = inflammatory cells roughly equal to melanocytes; 3 = predominantly inflammatory cells).

Lymphocytes infiltration within the tumor is graded on a scale of 0-3 (0 = absence of lymphocytes in the tumor; 1 = < 5 lymphocyte per high power field (HPF); 2 = >5 and < 20 lymphocytes per HPF 3 = > 20 lymphocytes per HPF).

The IHC (immunohistochemistry) scoring grade ) CD8, CD68) is held performed on a scale of 0 to 4:
0= No cells are detected
1= Very few cells are detected (1-5 per X10 magnification)
2= Few cells are detected (6-15 per X10 magnification)
3= Moderate number of cells are detected (16-50 per X10 magnification)
4= Many cells are detected (more than 50 per X10 magnification)

## Claims

1. A microbial consortium comprising two or more microorganisms for use in a method of inducing a pro-inflammatory effect and treating cancer in a human subject,
wherein said two or more microorganisms are selected from *Oenococcus oeni* species, *Clostridium cellulovorans* species, *Cetobacterium somerae* species, *Corynebacterium glyciniphilum* species *and Clostridium tyrobutyricum* species,
further wherein the cancer is selected from breast cancer, lung cancer, pancreatic cancer, bladder cancer, melanoma, kidney cancer, head and neck cancer, and lymphoma, and
further wherein the microbial consortium is administered in combination with a checkpoint inhibitor that is an anti- PD-1 inhibitor.

2. The microbial consortium for use of claim 1, wherein said two or more microorganisms are present at low-abundance in a microbiome of a reference human subject diagnosed with cancer, wherein said microorganism present at low-abundance has an average relative abundance of less than 0.5% in the microbiome of said reference human subject.

3. The microbial consortium for use of claim 1 or 2, comprising three or more microorganisms selected from *Oenococcus oeni* species, *Clostridium cellulovorans* species, *Cetobacterium somerae* species, *Corynebacterium glyciniphilum* species *and Clostridium tyrobutyricum* species.

4. The microbial consortium for use of any one of claims 1 to 3, wherein said two or more microorganisms are capable of modulating a pro-inflammatory cytokine.

5. The microbial consortium for use of claim 4, wherein the pro-inflammatory cytokine comprises at least one of interleukin-1 (IL-1), IL-1β, IL2, IL-6, IL8, IL-12, IL17, IL-18, IL22, IL23, tumor necrosis factor alpha (TNF-α), interferon gamma (IFNγ), and granulocyte-macrophage colony stimulating factor (GM-CSF).

6. The microbial consortium for use of any one of claims 1 to 5, wherein said cancer is a solid cancer.

## Patentansprüche

1. Mikrobielles Konsortium, das zwei oder mehr Mikroorganismen umfasst, für die Verwendung in einem Verfahren zum Induzieren einer proinflammatorischen Wirkung und für die Behandlung von Krebs bei einem menschlichen Subjekt,
wobei die zwei oder mehr Mikroorganismen aus *Oenococcus oeni-Arten, Clostridium cellulovorans-Arten, Cetobacterium somerae-Arten, Corynebacterium glyciniphilum-Arten* und *Clostridium tyrobutyricum-Arten* ausgewählt sind,
wobei ferner der Krebs aus Brustkrebs, Lungenkrebs, Bauchspeicheldrüsenkrebs, Blasenkrebs, Melanom, Nierenkrebs, Kopf- und Halskrebs und Lymphom ausgewählt ist und
wobei ferner das mikrobielle Konsortium in Kombination mit einem Checkpoint-Inhibitor verabreicht wird, der ein Anti-PD-1-Inhibitor ist.

2. Mikrobielles Konsortium für die Verwendung nach Anspruch 1, wobei die zwei oder mehr Mikroorganismen in geringer Häufigkeit in einem Mikrobiom eines menschlichen Referenzsubjekt vorhanden sind, bei dem Krebs diagnostiziert wurde, wobei der Mikroorganismus, der in geringer Häufigkeit vorhanden ist, eine durchschnittliche relative Häufigkeit von weniger als 0,5 % in dem Mikrobiom des menschlichen Referenzsubjekts aufweist.

3. Mikrobielles Konsortium für die Verwendung nach Anspruch 1 oder 2, das drei oder mehr Mikroorganismen umfasst, die aus *Oenococcus oeni-Arten, Clostridium cellulovorans-*Arten, *Cetobacterium somerae-Arten, Corynebacterium glyciniphilum-Arten* und *Clostridium tyrobutyricum-Arten* ausgewählt sind.

4. Mikrobielles Konsortium für die Verwendung nach einem der Ansprüche 1 bis 3, wobei die zwei oder mehr Mikroorganismen in der Lage sind, ein proinflammatorisches Zytokin zu modulieren.

5. Mikrobielles Konsortium für die Verwendung nach Anspruch 4, wobei das proinflammatorische Zytokin wenigstens eines von Interleukin-1 (IL-1), IL-1β, IL2, IL-6, IL8, IL-12, IL17, IL-18, IL22, IL23, Tumornekrosefaktor alpha (TNF-α), Interferon gamma (IFN-γ) und Granulozyten-Makrophagen-kolonienstimulierendem Faktor (GM-CSF) umfasst.

6. Mikrobielles Konsortium für die Verwendung nach einem der Ansprüche 1 bis 5, wobei der Krebs ein solider Krebs ist.

## Revendications

1. Consortium microbien comprenant deux ou plus de deux microorganismes à utiliser dans une méthode pour provoquer un effet pro-inflammatoire et traiter un cancer chez un sujet humain, dans lequel lesdits deux ou plus de deux microorganismes sont sélectionnés parmi l'espèce *Oenococcus oeni,* l'espèce *Clostridium cellulovorans,* l'espèce *Cetobacterium somerae,* l'espèce *Corynebacterium glyciniphilum,* et l'espèce *Clostridium tyrobutyricum,*
dans lequel en outre le cancer est sélectionné parmi le cancer du sein, le cancer du poumon, le cancer du pancréas, le cancer de la vessie, un mélanome, le cancer du rein, le cancer de la tête et du cou, et un lymphome, et
dans lequel en outre le consortium microbien est administré en association avec un inhibiteur de point de contrôle qui est un inhibiteur anti-PD-1.

2. Consortium microbien à utiliser de la revendication 1, dans lequel lesdits deux ou plus de deux microorganismes sont présents à faible abondance dans un microbiome d'un sujet humain de référence chez qui un cancer a été diagnostiqué, dans lequel ledit microorganisme présent à faible abondance a une abondance relative moyenne inférieure à 0,5 % dans le microbiome dudit sujet humain de référence.

3. Consortium microbien à utiliser de la revendication 1 ou 2, comprenant trois ou plus de trois microorganismes sélectionnés parmi l'espèce *Oenococcus oeni,* l'espèce *Clostridium cellulovorans,* l'espèce *Cetobacterium somerae,* l'espèce *Corynebacterium glyciniphilum,* et l'espèce *Clostridium tyrobutyricum.*

4. Consortium microbien à utiliser de l'une quelconque des revendications 1 à 3, dans lequel lesdits deux ou plus de deux microorganismes sont capables de moduler une cytokine pro-inflammatoire.

5. Consortium microbien à utiliser de la revendication 4, dans lequel la cytokine pro-inflammatoire comprend au moins un élément parmi interleukine-1 (IL-1), IL-1β, IL2, IL-6, IL8, IL-12, IL17, IL-18, IL22, IL23, facteur de nécrose tumorale alpha (TNF-α), interféron gamma (IFNγ), et facteur de stimulation des colonies granulocyte-macrophage (GM-CSF).

6. Consortium microbien à utiliser de l'une quelconque des revendications 1 à 5, dans lequel ledit cancer est un cancer solide.
